# EUROPEAN PATENT SPECIFICATION

(11) **EP 3 963 050 B1**
(45) Date of publication and mention of the grant of the patent: **13.09.2023**
(21) Application number: 20721612.8
(22) Date of filing: 30.04.2020
(51) Int. Cl.: C12N 5/071

(54) **PREPARATION OF HUMAN ALLOGENEIC LIVER-DERIVED PROGENITOR CELLS**
HERSTELLUNG VON HETEROLOGEN, AUS MENSCHLICHER ERWACHSENENLEBER GEWONNENEN VORLÄUFERZELLEN
PRÉPARATION DE CELLULES PROGÉNITRICES ADULTES HUMAINES HÉTÉROLOGUES DÉRIVÉES DU FOIE

(30) Priority: 30.04.2019 EP 19172076
(43) Date of publication of application: 09.03.2022
(73) Proprietor: Cellaïon SA, 1435 Mont-Saint-Guibert (BE)
(72) Inventor: STRAGIER, Patrick, 4053 Embourg (BE); PINXTEREN, Jozef, 9800 Bachte-Maria-Leerne (BE); DELTOUR, Elodie, 1410 Waterloo (BE); BOVY, Thierry, 4101 Jemeppe sur Meuse (BE)
(74) Representative: Brantsandpatents bv
(86) International application number: PCT/EP2020/061987
(87) International publication number: WO 2020/221843

(56) References cited:
- EP-A1- 1 969 118
- WO-A1-2016/030525
- BR-A2- PI0 620 049
- MARIA BEATRIZ HERRERA ET AL: "Isolation and characterization of a stem cell population from adult human liver", STEM CELLS,, vol. 24, no. 12, 1 January 2006 (2006-01-01), pages 2840-2850, XP008146144, ISSN: 1066-5099, DOI: 10.1634/STEMCELLS.2006-0114
- SASAKI KAZUNORI ET AL: "Proliferation of Hepatocyte Progenitor Cells Isolated From Adult Human Livers in Serum-Free Medium", CELL TRANSPLANTATION, SAGE, US, vol. 17, no. 10-11, 1 October 2008 (2008-10-01), pages 1221-1230, XP008166809, ISSN: 0963-6897, DOI: 10.3727/096368908787236666
- NAJIMI MUSTAPHA ET AL: "Adult-derived human liver mesenchymal-like cells as a potential progenitor reservoir of hepatocytes?", CELL TRANSPLANTATION, SAGE, US, vol. 16, no. 7, 1 January 2007 (2007-01-01), pages 717-728, XP008130765, ISSN: 0963-6897
- KATRIN ZEILINGER ET AL: "Cell sources for in vitro human liver cell culture models", EXPERIMENTAL BIOLOGY AND MEDICINE, vol. 241, no. 15, 24 July 2016 (2016-07-24), pages 1684-1698, XP055621901, GB ISSN: 1535-3702, DOI: 10.1177/1535370216657448

## Description

### BACKGROUND OF THE INVENTION

The present invention relates to human allogeneic liver-derived progenitor cells (HALPC or HALPCs) and their manufacture. These cells are also sometimes referred to as human adult liver-derived progenitor cells, or as heterologous human adult liver-derived progenitor cells (HHALPC or HHALPs).

The liver is a key organ in the regulation of body homeostasis and is the site of many vital metabolic pathways. Impairment of only one protein within a complex metabolic pathway could be highly deleterious. The large presence of important liver enzymes substantially increases the risk occurrence of diverse liver diseases. Altogether, 200 different inborn errors of liver metabolism exist, affecting 1 child over 2500 live births. Current treatments, and long-term management, are not efficient enough. Orthotopic liver transplantation (OLT) is highly intrusive, irreversible, limited by shortage of donor grafts and demands state-of-art surgery. Liver cell transplantation (LCT) may exert only short-to-medium term efficacy due to the quality of hepatocyte preparations.

The use of stem or progenitor cells, in particular liver progenitor cells has been identified in the literature using liver tissues from different organisms, as well as in fetal or adult liver tissues (Schmelzer E et al., 2007; Sahin MB et al., 2008; Azuma H et al., 2003; Herrera MB et al., 2006; Najimi M et al., 2007; Darwiche H and Petersen BE, 2010; Shiojiri N and Nitou M, 2012; Tanaka M and Miyajima A, 2012). Such cells are believed to potentially provide, following the exposure to hepatogenic stimuli in vitro and/or after in vivo administration, cells with morphological and functional features typically associated to hepatic differentiation such as phase I/II enzymatic activities.

These liver progenitor cells or hepatocyte-like cells that are generated from them can be used in cellular transplantation as well as for drug testing in the development of new drugs since they represent a surrogate for primary human hepatocytes in drug metabolism and pharmacological or toxicological in vitro screening (Dan YY, 2012; Hook LA, 2012).

WO 2016/030525 and WO 2017/149059 disclose specific cell culture conditions allowing the obtention of human adult liver-derived progenitor cells (HALPC) with specific expression profile and improved biological features. For example, WO2016/030525 describes the use of basal media formulations such as William's medium E, Dulbecco's Modified Eagle's Medium (DMEM) preferably complemented with mammalian plasma or sera in particular fetal bovine (calf) serum (i.e. FCS or FBS) in a process of culturing said cells. It is also described in WO 2016/030525, that besides providing nutrients and/or growth promoters, the liquid culture media with addition of bovine, human or other animal serum may also promote the growth/adherence or the elimination/detachment of specific cell types.

It is an object of the present invention to provide an efficient method of manufacturing human allogeneic liver-derived progenitor cells. A further object is to provide a method for producing human allogeneic liver-derived progenitor cells with high productivity, and which allows for commercially attractive batch processing times. A yet further object is to provide a stable process for manufacturing human allogeneic liver-derived progenitor cells, wherein the process ensures high yields of cells having a consistent quality, in line with GMP criteria.

### SUMMARY OF THE INVENTION

The invention relates to human liver-derived progenitor cells, in particular human allogeneic liver progenitor cells (HALPC), also referred to as heterologous human adult liver-derived progenitor cells (HHALPC), or human adult liver-derived progenitor cells (HALPC), and their manufacture.

In one aspect, the invention relates to a process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension under conditions that cause the emergence of a population of cells having an elongated shape and mesenchymal morphology;
(c) culturing the cell population emerged in step (b) under conditions that cause its expansion;
(d) harvesting the expanded cell population obtained in step (c);

wherein the cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and optionally express at least one hepatic marker and/or exhibit a liver-specific activity;
wherein the culturing conditions of step (b) include the use of a xeno- and serum-free culture medium comprising purified native or recombinant human serum albumin.

In a further aspect, the xeno- and serum-free culture medium is further characterized in that it contains less than 30 ng/mL of epidermal growth factor (EGF).

In yet a further aspect, step (c) of the process also includes the use of xeno-and serum-free culture medium comprising purified native or recombinant human serum albumin.

In another aspect, the invention relates to isolated populations of human allogeneic liver-derived progenitor cells (HALPCs) obtainable by said process. Further, the invention relates to said HALPCs for use in the treatment of a liver disease.

### DESCRIPTION OF THE DRAWINGS

Figure 1 depicts the morphology of emerged HALPCs (P0) cultivated in serum-free and xeno-free medium A (seeding density 66,666 cells/cm²), as visualized by phase contrast microscopy (100-fold magnification).

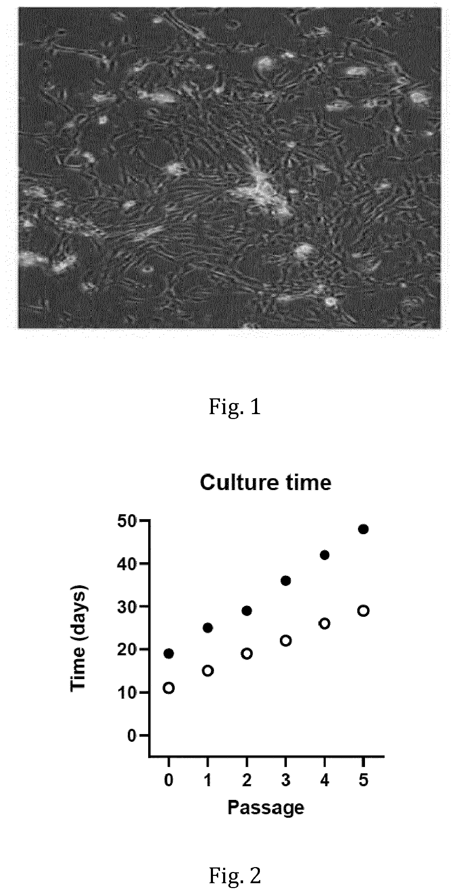

Figure 2 is a graphical comparison of the overall culture time from emergence P0 and subsequent to expansion passages 1 to 5 for a production run conducted with a xeno-and serum-free culture medium A (depicted in the graph as unfilled circles) compared to a reference serum-containing culture medium containing foetal bovine serum (FCS) (depicted in the graph as filled or solid circles). The overall culture time to P5 for the serum culture medium process culminates is higher at all passages, culminating in a total culture time of 48 days, where in comparison, a total time of 29 days is observed for the production with serum- and xeno-free medium A.
Figure 3 is a graphical comparison of the theoretical output yield per seed for the production of HALPCs in a reference serum-containing culture medium, compared to the theoretical output yield per seed for a production using xeno-free and serum-free culture medium A. A difference of almost 50 times higher theoretical yield can be determined for the process conducted in the xeno-free and serum-free culture medium compared to the reference culture media.
Figure 4 shows the level of clonal (left) and non-clonal (right) chromosomal aberrations occurring in otherwise comparable HALPCs prepared either according to the invention using a xeno- and serum-free culture medium or with a conventional process using a culture medium comprising bovine serum.
Figure 5 shows the secretion of HGF and PGE2 in otherwise comparable HALPCs prepared either according to the invention using a xeno- and serum-free culture medium or with a conventional process using a culture medium comprising bovine serum.

### DETAILED DESCRIPTION OF THE INVENTION

In one aspect, the present invention relates to a process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension under conditions that cause the emergence of a population of cells having an elongated shape and mesenchymal morphology;
(c) culturing the cell population emerged in step (b) under conditions that cause its expansion;
(d) harvesting the expanded cell population obtained in step (c);

wherein said cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and that optionally express at least one hepatic marker and/or exhibit a liver-specific activity;
wherein the culturing conditions of step (b) include the use of a xeno- and serum-free culture medium comprising purified native or recombinant human serum albumin.

In a further aspect, the present invention relates to a process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension under conditions that cause the emergence of a population of cells having an elongated shape and mesenchymal morphology;
(c) culturing the cell population emerged in step (b) under conditions that cause its expansion;
(d) harvesting the expanded cell population obtained in step (c);

wherein said cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and that optionally express at least one hepatic marker and/or exhibit a liver-specific activity;
wherein the culturing conditions of step (b) include the use of a xeno- and serum-free culture medium comprising purified native or recombinant human serum albumin, and wherein the xeno- and serum-free culture medium is further characterized in that it contains less than 30 ng/mL of epidermal growth factor (EGF).

The human allogeneic liver-derived progenitor cells obtained in step (c) or (d) may optionally be characterized in that they express at least one mesenchymal marker selected from CD90, CD44, CD73, CD13, CD140b, CD29, vimentin and α-smooth muscle actin (ASMA), and in that they secrete HGF; in addition, they may also secrete PGE2.

In one of the preferred embodiments, the method of the invention further comprises the step (e) of establishing that the cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and that optionally express at least one hepatic marker and/or exhibit a liver-specific activity.

In a further embodiment, the human allogeneic liver-derived progenitor cells obtained in step (c) or (d) are positive for α-smooth muscle actin (ASMA), CD140b and optionally albumin (ALB); and negative for Cytokeratin-19 (CK-19).

In a further aspect, the present invention relates to a process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension under conditions that cause the emergence of a population of cells having an elongated shape and mesenchymal morphology;
(c) culturing the cell population emerged in step (b) under conditions that cause its expansion;
(d) harvesting the expanded cell population obtained in step (c); and
(e) establishing that the cells of the expanded cell population obtained in step (c) coexpress at least one mesenchymal marker selected from CD90, CD44, CD73, CD13, CD140b, CD29, vimentin and alpha-smooth muscle actin (ASMA) with one or more hepatic marker selected from alphafetoprotein (AFP), alpha-1 antitrypsin, HNF-4 and MRP2 transporter and optionally the hepatic marker albumin;
wherein the culturing conditions of step (b) include the use of a xeno- and serum-free culture medium comprising purified native or recombinant human serum albumin.

In a further aspect, the present invention relates to a process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension under conditions that cause the emergence of a population of cells having an elongated shape and mesenchymal morphology;
(c) culturing the cell population emerged in step (b) under conditions that cause its expansion;
(d) harvesting the expanded cell population obtained in step (c); and
(e) establishing that the cells of the expanded cell population obtained in step (c) coexpress at least one mesenchymal marker selected from CD90, CD44, CD73, CD13, CD140b, CD29, vimentin and alpha-smooth muscle actin (ASMA) with one or more hepatic marker selected from alphafetoprotein (AFP), alpha-1 antitrypsin, HNF-4 and MRP2 transporter and optionally the hepatic marker albumin;
wherein the culturing conditions of step (b) include the use of a xeno- and serum-free culture medium comprising purified native or recombinant human serum albumin, and wherein the xeno- and serum-free culture medium is further characterized in that it contains less than 30 ng/mL of epidermal growth factor (EGF).

Step (a) according to the process of the present invention comprises the provision of a suspension of primary liver cells obtained from a human liver.

Sub-steps involved in obtaining the primary liver cell suspension (which may be referred to herein also as liver cell suspension, or abbreviated as LCS) from a human liver may include the following: loosening of intercellular tight junctions, the liver digestion, the liver disruption, filtration, the liver cell separation by centrifugation and optionally, the cryopreservation of the final liver cell suspensions.

The disassociating step of a human (adult) liver or a part thereof to form a population of primary liver cells involves obtaining a liver, or a part thereof that comprises, together with fully differentiated hepatocytes, an amount of primary cells that can be used for producing liver progenitor or stem cells.

As used herein, the term "liver progenitor cell" refers to an unspecialized and proliferation-competent cell which is produced by culturing cells that are isolated from liver and which or the progeny of which can give rise to at least one relatively more specialized cell type. A liver progenitor cell gives rise to descendants that can differentiate along one or more lineages to produce increasingly more specialized cells (but preferably hepatocytes or hepato-active cells), wherein such descendants may themselves still be progenitor cells, or even to produce terminally differentiated liver cells (e.g. fully specialized cells, in particular cells presenting morphological and functional features similar to those of primary human hepatocytes). The term "stem cell" refers to a progenitor cell capable of self-renewal, i.e., can proliferate without differentiation, whereby the progeny of a stem cell or at least part thereof substantially retains the unspecialized or relatively less specialized phenotype, the differentiation potential, and the proliferation competence of the mother stem cell. The term encompasses stem cells capable of substantially unlimited self-renewal, i.e., wherein the capacity of the progeny or part thereof for further proliferation is not substantially reduced compared to the mother cell, as well as stem cells which display limited self-renewal, i.e., wherein the capacity of the progeny or part thereof for further proliferation is demonstrably reduced compared to the mother cell.

As mentioned, the term "adult human liver-derived progenitor cells" is used synonymously with "human adult liver-derived progenitor cells", "heterologous human adult liver-derived progenitor cells" or "human allogeneic liver progenitor cells", abbreviated as "HHALPC" or "HHALPCs" or "HALPC" or "HALPCs". These cells represent a specific type of human liver-derived progenitor cells, obtainable as described herein. Currently preferred by the inventors is the term, "human allogeneic liver progenitor cells".

Based on the ability to give rise to diverse cell types, a progenitor or stem cell may be usually described as totipotent, pluripotent, multipotent or unipotent. A single "totipotent" cell is defined as being capable of growing, i.e. developing, into an entire organism. A "pluripotent" cell is not able of growing into an entire organism, but is capable of giving rise to cell types originating from all three germ layers, i.e., mesoderm, endoderm, and ectoderm, and may be capable of giving rise to all cell types of an organism. A "multipotent" cell is capable of giving rise to at least one cell type from each of two or more different organs or tissues of an organism, wherein the said cell types may originate from the same or from different germ layers, but is not capable of giving rise to all cell types of an organism. A "unipotent" cell is capable of differentiating to cells of only one cell lineage.

The liver or part thereof is obtained from a "subject", "donor subject" or "donor", interchangeably referring to a vertebrate animal, preferably a mammal, more preferably a human. A part of a liver can be a tissue sample derived from any part of the liver and may comprise different cell types present in the liver. The term "liver" refers to liver organ. The term "part of liver" generally refers to a tissue sample derived from any part of the liver organ, without any limitation as to the quantity of the said part or the region of the liver organ where it originates. Preferably, all cell types present in the liver organ may also be represented in the said part of liver. The quantity of the part of liver may at least in part follow from practical considerations to the need to obtain enough primary liver cells for reasonably practicing the method of the invention. Hence, a part of liver may represent a percentage of the liver organ (e.g. at least 1%, 10%, 20%, 50%, 70%, 90% or more, typically w/w). In other nonlimiting examples, a part of liver may be defined by weight (e.g. at least 1 g, 10 g, 100 g, 250 g, 500 g, or more). For example, a part of liver may be a liver lobe, e.g., the right lobe or left lobe, or any segment or tissue sample comprising a large number of cells that is resected during split liver operation or in a liver biopsy.

The cells used according to the invention are isolated from human liver or part of a human liver. More preferably, the liver progenitor cell or stem cell is isolated from an adult human liver or a part thereof. The term "adult liver" refers to liver of subjects that are post-natal, i.e. any time after birth, preferably full term, and may be, e.g., at least 1 day, 1 week, 1 month or more than 1 month of age after birth, or at least 1, 5, 10 years or more. Hence, an "adult liver", or mature liver, may be found in human subjects who would otherwise be described in the conventional terms of "neonate", "infant", "child", "adolescent", or "adult". A skilled person will appreciate that the liver may attain substantial developmental maturity in different time postnatal intervals. In one preferred, embodiment, the cells are isolated from a neonate human liver, or part of the liver (e.g. a liver lobe).

A donor subject may be living or dead, as determined by art-accepted criteria, such as, for example, the "heart-lung" criteria (usually involving an irreversible cessation of circulatory and respiratory functions) or the "brain death" criteria (usually involving an irreversible cessation of all functions of the entire brain, including the brainstem). Harvesting may involve procedures known in the art, such as, for example, biopsy, resection or excision.

A skilled person will appreciate that at least some aspects of harvesting liver or part thereof from donor subjects may be subject to respective legal and ethical norms. By means of example and not limitation, harvesting of liver tissue from a living human donor may need to be compatible with sustenance of further life of the donor.

Accordingly, only a part of liver may typically be removed from a living human donor, e.g., using biopsy or resection, such that an adequate level of physiological liver functions is maintained in the donor.

Liver or part thereof may be obtained from a human donor, who has sustained circulation, e.g., a beating heart, and sustained respiratory functions, e.g., breathing lungs or artificial ventilation. Subject to ethical and legal norms, the donor may need to be or need not be brain dead (e.g., removal of entire liver or portion thereof, which would not be compatible with further survival of a human donor, may be allowed in brain dead human beings). Harvesting of liver or part thereof from such donors is advantageous, since the tissue does not suffer substantial anoxia (lack of oxygenation), which usually results from ischemia (cessation of circulation).

Alternatively, liver or part thereof may be obtained from a human donor, who at the time of harvesting the tissue has ceased circulation, e.g., has a non-beating heart, and/or has ceased respiratory functions, e.g., has non-breathing lungs and no artificial ventilation. While liver or part thereof from these donors may have suffered at least some degree of anoxia, viable progenitor or stem cells can also be isolated from such tissues. Liver or part thereof may be harvested within about 24h after the donor's circulation (e.g., heart-beat) ceased, e.g., within about 20h, e.g., within about 16h, more preferably within about 12h, e.g., within about 8h, even more preferably within about 6h, e.g., within about 5h, within about 4h or within about 3h, yet more preferably within about 2h, and most preferably within about 1h, such as, within about 45, 30, or 15 minutes after the donor's circulation (e.g., heart-beat) ceased.

The harvested tissues may be cooled to about room temperature, or to a temperature lower than room temperature, but usually freezing of the tissue or parts thereof is avoided, especially where such freezing would result in nucleation or ice crystal growth. For example, the tissue may be kept at any temperature between about 1°C and room temperature, between about 2°C and room temperature, between about 3°C and room temperature or between about 4°C and room temperature, and may be advantageously be kept at about 4°C. The tissue may also be kept "on ice" as known in the art. The tissue may be cooled for all or part of the ischemic time, i.e., the time after cessation of circulation in the donor. That is, the tissue can be subjected to warm ischemia, cold ischemia, or a combination of warm and cold ischemia. The harvested tissue may be so kept for, e.g., up to 48h before processing, preferably for less than 24h, e.g., less than 16h, more preferably for less than 12h, e.g., less than 10h, less than 6h, less than 3h, less than 2h or less than 1 h.

The harvested tissue may advantageously be kept in, e.g., completely or at least partly submerged in, a suitable medium and/or may be perfused with the suitable medium, before further processing of the tissue. A skilled person is able to select a suitable medium which can support the survival of the cells of the tissue during the period before processing.

Isolation of primary liver cells from a liver or part of a liver may be performed according to methods known in the art, for example as described in EP1969118, EP3039123, EP3140393 or WO2017149059 (see Example 1).

A suspension of primary liver cells is first obtained from disassociating of liver or part thereof. The term "disassociating" as used herein refers to partly or completely disrupting the cellular organization of a tissue or organ, i.e. partly or completely disrupting the association between cells and cellular components of a tissue or organ to obtain a suspension of cells from the said tissue or organ. The suspension may comprise solitary or single cells, as well as cells physically attached to form clusters or clumps of two or more cells. Disassociating preferably does not cause - or causes as small as possible - reduction in cell viability. A suitable method for disassociating liver or part thereof to obtain a population (suspension) of primary cells therefrom may be any method well known in the art, including but not limited to, enzymatic digestion, mechanical separation, filtration, centrifugation and combinations thereof. In particular, the method for disassociating liver or part thereof may comprise enzymatic digestion of the liver tissue to release liver cells and/or mechanical disruption or separation of the liver tissue to release liver cells. Small, thin fragments of liver tissues that are obtained by a liver biopsy may be used directly for pursuing cell culture according to the following Step (c) without enzymatic or mechanical disruption.

Methods for disassociating liver or part thereof as above are documented in the literature, such as the widely used collagenase perfusion technique in two or more steps, which has been variously adapted and modified for performing it with whole livers or segments of liver. The liver tissue is perfused with a divalent cation-free buffer solution, preheated at 37°C, containing a cation-chelating agent (e.g. EDTA or EGTA). Buffer solutions can comprise salt solutions (e.g. HEPES, Williams E medium) or any other balanced salt solution that can also include salts such as NaCl and KCl, among others. This leads to disruption of the desmosomal structures that hold cells together.

The tissue is then perfused with the buffer solution containing divalent cation(s), such as Ca²⁺ and Mg²⁺, and matrix-degrading enzymes that act to digest the tissue. The primary liver cells are usually released by gentle mechanical disruption and/or pressing through filters, to mechanically complete the cell dissociation process. Such filters may have sieve sizes that allow passage of cells through about 0.1mm, 0.25mm, 0.50mm, 1mm or more. A succession of filters with progressively smaller sieve sizes may be used to gradually disassociate the tissue and release cells. The dissociated cells are rinsed with a buffer containing protease inhibitor, serum and/or plasma to inactivate collagenase and other enzymes used in the perfusion process, and then separated from the mixture by pelleting them with low speed centrifugation (e.g. at between 10 x g and 500 x g). Most of, if not all, viable cells can be pelleted, while dead cells and cell debris are substantially eliminated and subsequently are washed with ice-cold buffer solution to purify the cell suspension. The number and quality of the primary liver cells can vary depending on the quality of the tissue, the compositions of different solutions that are used, and the type and concentration of enzyme. The enzyme is frequently collagenase but also pronase, trypsin, hyaluronidase, thermolysin, and combinations thereof can be used. Collagenase may consist of a poorly purified blend of enzymes and/or exhibit protease activity, which may cause unwanted reactions affecting the quality and quantity of viable cells that can in turn be avoided by selecting enzyme preparations of sufficient purity and quality. Other methods of harvesting primary liver cells may exclude enzymatic digestion techniques and may involve perfusing liver with solutions containing sucrose followed by mechanical disruption.

The population of primary cells as defined and obtained herein by disassociating liver or part thereof may typically be heterogeneous, i.e., it may comprise cells belonging to one or more cell types belonging to any liver-constituting cell type, including progenitor or stem cells, that may have been present in liver parenchyma and/or in the liver non-parenchymal fraction. As used herein, the term "primary cell" includes cells present in a suspension of cells obtained from a tissue or organ of a subject, e.g. liver, by disassociating cells present in such explanted tissue or organ with appropriate techniques.

Exemplary liver-constituting cell types include but are not limited to hepatocytes, cholangiocytes (bile duct cells), Kupffer cells, hepatic stellate cells (Ito cells), oval cells and liver endothelial cells. The above terms have art-established meanings and are construed broadly herein as encompassing any cell type classified as such.

The term "hepatocyte" encompasses epithelial and parenchymal liver cells, including but not limited to hepatocytes of different sizes or ploidy (e.g., diploid, tetraploid, octaploid). A primary cell population may comprise hepatocytes in different proportions (0.1%, 1%, 10%, or more of total cells), according to the method of disassociating liver and/or any method for fractioning or enriching the initial preparation for hepatocytes and/or other cell types on the basis of physical properties (dimension, morphology), viability, cell culture conditions, or cell surface marker expression by applying any suitable technique.

The population of primary cells as defined and obtained herein by disassociating liver (or part of it) can be used immediately for establishing cell cultures as suspensions of fresh primary liver cells. The human liver cell suspension (LCS) consists of human primary liver cells in dispersion, isolated from human-donated liver.

Alternatively, the obtained primary human liver cells may be stored as cryopreserved preparations using common technologies for their long-term preservation. In one embodiment, the primary human liver cell suspension used in the manufacturing process according to the present invention may be obtained and used as a cryopreserved product (stored at ≤-130°C), which is thawed (e.g. at 37°C in a water bath) and washed in a thawing solution before use.

In some embodiments, the concentration of liver cells in the liver cell suspension used in the present process may be greater than 7.5×10⁶ cells/mL, for example 10×10⁶ cells/mL, or 20×10⁶ cells/mL.

Step (b) of the process according to the present invention comprises culturing the primary liver cells comprised in the provided human primary liver cell suspension under conditions that cause the emergence of a population of cells, i.e. the human allogeneic liver-derived progenitor cells, or HALPC having an elongated shape and mesenchymal morphology.

The primary liver cells may be cultured directly onto a fully synthetic support (e.g. plastic or any polymeric substance) or a synthetic support pre-coated with feeder cells, protein extracts, or any other material of biological origin that allow the adherence and the proliferation of similar primary cells and the emergence of a population of allogeneic liver progenitor cells having the desired markers, and morphology.

The primary liver cells are cultured in a cell culture medium sustaining their adherence and the proliferation and/or emergence of a homogenous cell population. According to a preferred embodiment of the present invention, a xeno- and serum-free culture medium comprising purified native or recombinant human serum albumin is used in step (b), wherein said medium is further characterized in that it contains less than 30 ng/mL of epidermal growth factor, or alternatively, no epidermal growth factor (EGF). Also preferred are embodiments wherein the medium comprises epidermal growth factor at a concentration of not more than about 25 ng/mL, or not more than about 10 ng/mL, or not more than 5 ng/mL, respectively.

As understood herein, the cell medium is a liquid medium which supports the survival and/or growth of the isolated liver progenitor cells. The liquid culture medium may be added to the culturing environment or system before, together with or after the introduction of the cells thereto. The term "cell medium" or "cell culture medium" or "medium" may generally refer to an aqueous liquid or gelatinous substance comprising nutrients which can be used for maintenance or growth of cells. In the context of the present invention, a liquid medium is used.

According to the present invention, the cell culture medium as used in step (b) and/or subsequent steps such as step (c) is serum-free. As per its conventional definition, "serum" is obtained from a sample of whole blood by first allowing clotting to take place in the sample and subsequently separating the so formed clot and cellular components of the blood sample from the liquid component (serum) by an appropriate technique, typically by centrifugation. The medium used for culturing step (b), and preferably also in any one or all of the subsequent steps of the process such as step (c) is free of serum that is derived from any source, whether animal, and/or human. In other words, serum as such is absent. However, this definition does not exclude the presence of an individual component that has been isolated from serum, such as highly purified serum albumin.

The cell culture medium as used in step (b), and/or subsequent steps of the process such as step (c) is also xeno-free. The term 'xeno-free', as used herein, may be understood as `animal-component free' (ACF), and refers to a medium (or substrate) which contains no non-human animal-derived or sourced components. In other words, the cell culture medium does not contain any ingredients of animal origin, or ingredients which may have been produced using animal host cells or animal expression cultures. The term 'animal' as used herein is understood to be any non-human animal, e.g. non-human mammals, such as non-human primates, fetal, calf or adult bovine, horse, porcine, lamb, goat, dog, rabbit, mouse or rat.

In one specific embodiment, the culture medium is a chemically-defined medium, meaning that the medium comprises no human purified components derived from human serum, but only recombinant components, which are also preferably manufactured from non-animal expression cultures.

As said, although free of serum as such, the cell culture medium as used in step (b) of the process according to the invention, and/or subsequent steps of the process such as step (c) comprises, as an individual component, purified native or recombinant human serum albumin. In one particular embodiment, the cell culture medium as used in step (b) of the process according to the invention, and/or subsequent steps of the process such as step (c) comprises a recombinant human serum albumin.

In a further embodiment, the amount of purified native or recombinant human serum in the culture medium is no more than 0.2 wt.-%, or between 0.01% and 2 wt.-%.

In yet a further embodiment, the culture medium comprises a plant-(e.g. rice) expressed recombinant human serum albumin.

If recombinant human serum albumin, or any one or combination of recombinant proteins or biological factors are used or supplemented to the culture medium used in step (b) (or step (c)) of the process, preferably said proteins or factors are also xeno-free, i.e. manufactured or produced without the use of any animal host cell or culture conditions and without the use of animal components.

Lipids and lipid carriers may also be used to supplement the cell culture media. Such lipids and carriers can include, but are not limited to, sterols such as cholesterol, fatty acids (or derivatives, e.g. esters, of fatty acids) such as linoleic acid, omega-3 or omega-6 fatty acid. As used herein, such fatty acid derivatives are included within the scope of the expressions "fatty acid" and "fatty acids". In one of the preferred embodiments, at least some of the lipids are bound to or associated with the purified native, or recombinant human serum albumin featured in the culture media.

In one embodiment, the culture medium comprises cholesterol and one or more fatty acid derivatives.

In one embodiment, the xeno- and serum-free culture medium as defined above and used in the process according to the invention comprises lipids, preferably a lipid selected from one or more fatty acids, wherein the lipids are associated with the purified native or recombinant human serum albumin.

In one embodiment, the one or more lipids comprised in the culture medium and associated with the human serum albumin is a fatty acid, for example a saturated or unsaturated C13 to C22 fatty acid, or a derivative thereof such as an ester or a salt. In another embodiment, the fatty acid associated with the human serum albumin is selected from any one or combination of any one of myristic acid, pentadecanoic acid, palmitic acid, palmitoleic acid, stearic acid, oleic acid, linoleic acid, vaccenic acid, gamma-linolenic acid, alpha-linolenic acid, dihomogamma-linolenic acid, arachidic acid, 11-eicoenoic acid, eicosadienoic acid, arachidonic acid, eicosapentaenoic acid, behenic acid, erucic acid, docosapentaenocic acid, docosahexaenoic acid, meads' acid and nervonic acid. In further embodiments, the fatty acids comprised in the culture medium and associated with the native or recombinant human serum albumin are selected from any one or combination of myristic acid, linoleic acid, one or more linolenic acids, and eicosapentaenoic acid. In yet a further embodiment, the fatty acid comprised in the culture medium, and associated with a recombinant human serum albumin is selected from at least one or any combination of eicosapentaenoic acid, behenic acid, erucic acid, doscosatetraenoic acid, and docosapentaenoic acid. In yet another embodiment, the fatty acid comprised in the culture medium and associated with a recombinant human serum albumin is selected from one or combination of Mead's acid and nervonic acid.

The term 'associated with or bound to human serum albumin', either in its native form or as a recombinant molecule, refers to a lipid, or fatty acid which is bound to one or more binding sites on the albumin, for example via hydrophobic binding, or by electrostatic or hydrogen binding actions. Optionally, the lipid may be covalently attached or conjugated to the human serum albumin.

In a further embodiment, the lipid-associated human native or recombinant human serum albumin featured in the xeno- and serum-free culture medium of the present invention may be obtained or obtainable by pre-treatment of the human serum albumin with one or a mixture of any of the above defined fatty acids.

The cell culture medium used to culture the liver progenitor cells according to the present invention may include a mixture of growth factors for their appropriate growth, proliferation, maintenance of desired markers and/or biological activity, or long-term storage.

The cell culture medium as used in step (b) of the process according to the invention, and/or subsequent steps of the process such as step (c) may comprise one or more growth factors, but is characterized in that it contains less than 30 ng/mL of EGF, and optionally no EGF. Also preferred are embodiments according to which the medium comprises epidermal growth factor at a concentration of not more than about 25 ng/mL, or not more than about 10 ng/mL, or not more than 5 ng/mL, respectively.

The term "growth factor" as used herein refers to a biologically active substance which influences proliferation, growth, differentiation, survival and/or migration of various cell types, and may effect developmental, morphological and functional changes in an organism, either alone or when modulated by other substances. A growth factor may typically act by binding, as a ligand, to a receptor (e.g., surface or intracellular receptor) present in cells. A growth factor herein may be particularly a proteinaceous entity comprising one or more polypeptide chains. The term "growth factor" encompasses the members of the fibroblast growth factor (FGF) family, bone morphogenic protein (BMP) family, platelet derived growth factor (PDGF) family, transforming growth factor beta (TGF-beta) family, nerve growth factor (NGF) family, the epidermal growth factor (EGF) family, the insulin related growth factor (IGF) family, the hepatocyte growth factor (HGF) family, the interleukin-6 (IL-6) family (e.g. oncostatin M), hematopoietic growth factors (HeGFs), the platelet-derived endothelial cell growth factor (PD-ECGF), angiopoietin, vascular endothelial growth factor (VEGF) family, or glucocorticoids.

In another embodiment, the xeno- and serum-free culture medium according to the invention comprises less than 30 ng/mL of EGF, or preferably less than 10 ng/mL of EGF, or optionally no EGF, but comprises at least three other growth factors, selected from any one of the above listed growth factors.

In one embodiment, the culture medium according to the invention comprises less than 30 ng/mL of EGF, or optionally no EGF, but may comprise at least one growth factor selected from the group consisting a transforming growth factor beta (TGF-beta), a fibroblast growth factor (FGF) and a platelet-derived growth factor (PDGF), or a combination thereof.

In one embodiment, the growth factor used in the present method may be a human or a human recombinant growth factor.

In further embodiments, said cell-culture medium is characterized in that it contains less than 25 ng/mL, or less than 10 ng/mL or less than 5 ng/mL of any one of a human growth factor.

In another embodiment, the cell culture medium is free of an epidermal growth factor, i.e. does not comprise of any epidermal growth factors (EGF).

In further embodiments, said cell-culture medium is characterized in that it contains less than 25 ng/mL, or less than 10 ng/mL or less than 5 ng/mL of EGF. Preferably, the culture cell medium contains less than 10 ng/mL of epidermal growth factor.

As used herein, the term 'epidermal growth factor' or 'EGF' refers to a growth factor which binds to the receptor EGFR, and optionally may encompass not only the peptide EGF as such but any one or combination of peptides considered within the art as belonging to EGF-family of proteins. Said term may encompass both native (human), as well as recombinant human EGF.

In this context, and for the avoidance of doubt, the content of growth factors such as EGF with respect to the culture medium should be understood as referring to the composition of the respective culture medium before it is combined with any cells. It is clear that the cultured cells, in this case the HALPCs, may themselves secrete growth factors. In other words, in one embodiment of the invention, the culture medium is free of EGF before it is combined with the cells.

The culture medium used in the context of the present invention may include inorganic salts (in particular salts containing Na, K, Mg, Ca, Cl, P, Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g. glucose, pyruvate, e.g., sodium pyruvate, acetate, e.g., sodium acetate), etc.

The culture media used may also be further supplied with one or more further components. For example, additional supplements can be used to supply the cells with the necessary trace elements and substances for optimal growth and expansion. Such supplements include insulin, transferrin, selenium salts, and combinations thereof. Further antioxidant supplements may be added, e.g., β-mercaptoethanol. In one embodiment of the invention, the culture medium or media used in the context of the present invention is supplemented with an antioxidant selected from ascorbic acid, or a salt thereof. In a further embodiment, amino acids may be supplemented to the culture medium, for example, L-glutamine, which is known to be less stable when in solution.

Hormones can also be supplemented and may include, but are not limited to D-aldosterone, diethylstilbestrol (DES), dexamethasone, estradiol, hydrocortisone, insulin, prolactin, progesterone, somatostatin/human growth hormone (HGH), thyrotropin, thyroxine, and L-thyronine. Liver cells can also benefit from culturing with triiodithyronine, α-tocopherol acetate, and glucagon.

In one embodiment, the xeno- and serum-free culture medium used in the process according to the invention (e.g. in step (b), and/or also step (c)) comprises:
(i) a purified native or recombinant human transferrin, and wherein the human transferrin has optionally been pre-treated with an iron compound;
(ii) recombinant human insulin; and
(iii) a selenium compound, such as sodium selenite.

The amount of human transferrin (native or recombinant) in the xeno- and serum-free culture medium is preferably not more than about 0.1 wt.-%. In one embodiment, the amount of human transferrin featured in the culture medium is between 0.0001% and 0.1 wt.-%, such as between 0.001-wt.-% and 0.1.-wt%

The amount of recombinant human insulin in the xeno- and serum-free culture medium is preferably not more than about 0.05 wt.-%. In one embodiment, the amount of human recombinant insulin is between 0.001 wt.-% and 0.05 wt.-%.

In a further embodiment, the xeno- and serum-free culture medium used in the process according to the invention (i.e in step (b), and/or also step (c)) comprises:
(i) a recombinant human transferrin, wherein the human transferrin has optionally been pre-treated with an iron compound, and further optionally wherein the recombinant human transferrin is a plant-expressed recombinant human transferrin, e.g. a rice-expressed recombinant human transferrin;
(ii) recombinant human insulin; and
(iii) a selenium compound, such as sodium selenite.

In another embodiment, the xeno- and serum-free culture medium used in the process according to the invention (e.g. in step (b), and/or also step (c)) comprises about 0.01-2 wt.-% of a purified native or recombinant human serum albumin, no more than 30 ng/mL, or preferably no more than 10 ng/mL of an epidermal growth factor; and wherein said xeno- and serum-free culture medium further comprises:
(i) about 0.0001-0.1 wt.-% of a purified native or a recombinant human transferrin, and wherein the human transferrin has optionally been pre-treated with an iron compound;
(ii) about 0.001 to 0.05 wt.-% of a recombinant human insulin; and
(iii) a selenium compound, such as sodium selenite.

In another embodiment of the process, the xeno- and serum-free culture medium comprises human fibronectin and/or human vitronectin, such as recombinant human fibronectin and/or recombinant human vitronectin.

In another preferred embodiment, the culture medium comprises basic fibroblast growth factor (FGF2), also referred to as BFGF, FGF-2, FGFB, HBGF-2, fibroblast growth factor 2. If present, it is preferably used at a level of about 30 pg/mL or higher, such as in the range of about 30 to about 10,000 pg/mL. It may also be used at a level of about 100 pg/mL or higher, such as in the range of about 100 to about 10,000 pg/mL.

Also preferred are embodiments in which the culture medium comprises acidic fibroblast growth factor (FGF1) in addition to FGF2. If present, FGF1 may for example be used at a level of 5 pg/mL or higher, such as in the range of about 5 to about 500 pg/mL. Particularly useful is also the range of about 5 to about 100 pg/mL.

Further preferred are embodiments in which the culture medium further comprises granulocyte-colony stimulating factor (G-CSF), optionally at a level of about 1 to about 100 pg/mL. In one embodiment, the xeno- and serum-free culture medium comprises FGF2 at a level of about 100 to about 10,000 pg/mL, FGF1 at a level of about 5 to about 100 pg/mL, and G-CSF at a level of about 1 to about 100 pg/mL.

In yet another embodiment, the xeno- and serum-free culture medium comprises glucose, and wherein the glucose content is not higher than about 2.5 wt.-%, and optionally not higher than about 1 wt.-%. In a further embodiment, the xeno- and serum-free culture medium comprises glucose, wherein the glucose content is about 0.001 - 2.5 wt.-%, or optionally about 0.001-1 wt.-%.

As understood herein, the term wt.-% or weight percent is the weight of a component in a composition, expressed as a percentage of the total weight of the composition.

In yet a further embodiment, the xeno- and serum-free culture medium comprises glutamine or a salt thereof, or a compound capable of releasing glutamine or a salt thereof. A compound capable of releasing glutamine, or salt thereof may be a prodrug of glutamine.

In yet a further embodiment the xeno- and serum-free culture medium used in the culturing steps of the process according to the invention comprises:
(iv) inorganic salts, including salts of calcium, iron (II), iron (III), potassium, copper (II), magnesium, sodium, and zinc;
(v) amino acids, including L-alanine, L-arginine, L-asparagine, L-aspartic acid, L-cysteine, L-cystine, L-glutamine, L-glutamic acid, glycine, L-histidine, L-isoleucine, L-leucine, L-methionine, L-phenylalanine, L-proline, L-serine, L-threonine, L-tryptophane, L-tyrosine, and L-valine;
(vi) vitamins, including D(+)-biotin, D-calcium pantothenate, choline chloride, folic acid, myo-inositol, nicotinamide, pyridoxal, pyridoxine, riboflavin, thiamine, and vitamin B12; and optionally
(vii) DL-thioctic acid, hypoxanthine, linoleic acid, phenol red sodium, putrescine, pyruvate, and/or thymidine; and
wherein the pH of the culture medium is adjusted to pH 6.9-7.5.

The xeno- and serum-free culture medium may be prepared by combining the individual components, or it may be selected (and optionally adapted) from commercially available products based on the guidance provided herein. For example, commercially available xeno- and serum-free culture media that may potentially be used, optionally after adaptation according to the optional features and/or preferences disclosed herein, currently include, without limitation, PRIME-XV^{®} MSC Expansion XSFM (FUJIFILM Irvine Scientific) and StemMACS^{®} MSC Expansion kit XF (Miltenyi Biotech), including any GMP grade versions or equivalents thereof, such as MSC-Brew GMP Medium (Miltenyi Biotech). These media are currently also either free of EGF or contain EGF at a concentration of less than 30 ng/mL.

The seeding density used in the culturing of step (b) may optionally be in the range from about 5,000 to about 100,000 cells/cm², from about 10,000 to about 80,000 cells/cm², or from about 20,000 to about 66,666 cells/cm², respectively.

The culturing of primary liver cells as defined in step (b) leads to emergence and proliferation of liver progenitor cells in the culture and can be continued until liver progenitor or stem cells have proliferated sufficiently.

According to the invention, the cells are cultured for a period of time until emergence of a population of cells, i.e. the human allogeneic liver-derived progenitor cells, or HALPC having an elongated shape and mesenchymal morphology is observed.

In one embodiment, culturing is continued until the cell population has achieved a certain degree of confluence. The term "confluence" as used herein refers to a density of cultured cells in which the cells contact one another, covering substantially all of the surfaces available for growth (i.e., fully confluent). The degree of confluence may be determined by standard imaging (e.g. microscopy) and analysis methods commonly used in the art.

In one embodiment, the duration of step (b) may be selected such that at least 40 % confluence is reached. In other embodiments, step (b) is performed until about at least 50%, 70%, or at least 90% or more confluence is reached. In other embodiments, step (b) is performed until at least 80 % confluence is reached.

In yet another embodiment, the cells may be cultured in step (b) for at least 7 days, at least 10, or at least 12 days. Preferably, the emergence of the progenitor cells from the primary cell population in step (b), wherein at least 80% confluence is reached occurs within an average (mean) of 6 and 12 days, preferably within an average of 7 and 10 days.

It has been found by the inventors that the use of a xeno- and serum-free culture medium comprising purified native or recombinant human serum albumin, and wherein the xeno- and serum-free culture medium is further characterized in that it contains less than 30 ng/mL of an epidermal growth factor significantly reduces the duration of step (b) and the emergence of the HALPCs from the liver cell suspension. This time advantage may contribute to an overall shorter manufacturing time, and substantially decreased associated costs for preparation of the HALPCs.

The HALPC which emerge during step (b) have an elongated shape and mesenchymal morphology. The shape of these cells is fibroblast-like, supporting a mesenchymal-like profile which may be observed, for example, using phase-contrast microscopy. Elongate shape and mesenchymal profile of human mesenchymal stem cells are described in the art and general literature. The elongated shape and mesenchymal morphology of HALPC is shown in reference Figure 1, which depicts cultured HALPC visualized by phase contrast microscopy (100-fold magnification). Said cells may emerge and proliferate in the form of clusters/colonies layered on the culture support medium.

The HALPC are human mesenchymal liver progenitor cells derived from human adult livers and express both mesenchymal and hepatic markers. In addition to morphological evaluation, the liver progenitor cells emerging from step (b) may optionally be further characterized by technologies that allow detecting relevant markers already at this stage, for example as described in EP3140393 or WO2017149059. Among the technologies used for identifying such markers and measuring them as being positive or negative, Western Blot, Flow Cytometry immunocytochemistry, and ELISA are preferred since these allow marker detection at the protein level even with the low amount of liver progenitor cells that are available at this step.

The exact marker profile of the liver progenitor cells may to some extent depend on the desired cell subtype and other factors such as the microenvironment of the cells or their age. In general, the HALPCs prepared according to the invention (e.g. the cells obtained in step (c) or step (d)) have in common that they express at least one mesenchymal marker selected from CD90, CD44, CD73, CD13, CD140b, CD29, vimentin and alpha-smooth muscle actin (ASMA), and that they optionally express at least one hepatic marker and/or exhibit a liver-specific activity.

In one of the preferred embodiments, the HALPCs are characterized in that they express at least one mesenchymal marker selected from CD90, CD44, CD73, CD13, CD140b, CD29, vimentin and α-smooth muscle actin (ASMA), and in that they secrete hepatocyte growth factor (HGF), and optionally also prostaglandin E2 (PGE2). In a related embodiment, the HALPCs are positive for α-smooth muscle actin (ASMA), CD140b and optionally albumin (ALB), and negative for Cytokeratin-19 (CK-19).

In another preferred embodiment, the HALPCs as obtained in step (c) or (d) of the process according to the invention are characterized in that they express, or are positive for, CD90, CD73, vimentin and ASMA.

As said, the liver progenitor cells may be positive for at least one hepatic marker and at least one mesenchymal marker. They may have at least one liver-specific activity. For example, hepatic markers include but are not limited to, HNF-3B, HNF-4, CYP1A2, CYP2C9, CYP2E1, CYP3A4 and alpha-1 antitrypsin and may also include albumin (ALB). Mesenchymal markers include but are not limited to Vimentin, CD13, CD90, CD73, CD44, CD29, α-smooth muscle actin (ASMA) and CD140-b. Liver-specific activities include but are not limited to urea secretion, bilirubin conjugation, alpha-1-antitrypsin secretion and CYP3A4 activity.

In one optional embodiment, the process may comprise, directly subsequent to step (b), for example once a defined confluence of at least 40 % is reached, a further step of establishing that emerging cell population obtained in step (b) expresses at least one mesenchymal marker selected from CD90, CD44, CD73, CD13, CD140b, CD29, vimentin and alpha-smooth muscle actin (ASMA), and that they optionally express at least one hepatic marker and/or exhibit a liver-specific activity.

In another optional embodiment, the process may comprise, directly subsequent to step (b), for example once a defined confluence of at least 40 % is reached, a further step of establishing that emerging cell population obtained in step (b) co-expresses at least one mesenchymal marker selected from CD90, CD44, CD73, CD13, CD140b, CD29, vimentin and alpha-smooth muscle actin (ASMA) with one or more hepatic marker selected from alphafetoprotein (AFP), alpha-1 antitrypsin, HNF-4 and MRP2 transporter and optionally the hepatic marker albumin.

Step (c) of the process is culturing of the cell population emerged in step (b) under conditions that cause its expansion, and step (d) comprises the harvesting of said expanded cell population.

In step (c), the primary cells are cultured in a cell culture medium sustaining their adherence and the proliferation of a homogenous cell population that, following at least one passage, is progressively enriched for liver progenitor cells or stem cells. These liver progenitor cells can be rapidly expanded for generating sufficient cells for obtaining progeny having the desired properties for at least 2, 3, 4, 5 or more passages.

The term "passage" or "passaging" refers to the subculture of cells, which may be conducted by harvesting and re-seeding a cell culture or by replacing the culture medium, e.g. for the purpose of extending the life of the cells or expanding their number. The passage performed after the first period of growing the cells under adherent culture conditions in the expansion step (c) is herein referred to as "first passage" (or passage 1, P1) within the method of the invention. The cells may be passaged at least one time and preferably two or more times. Each passage subsequent to passage 1 is referred to herein with a number increasing by 1, e.g., passage 2, 3, 4, 5, or P1, P2, P3, P4, P5, etc. A passage may comprise the steps of (i) seeding or plating the cells obtained from step (b) or an earlier passage on to a suitable substrate for cell growth and expansion, culturing said cells under conditions to cause their expansion, and (ii) detaching and dissociating the cultured cells from the culture substrate and from each other.

In one embodiment, step (c) of the process according to the invention comprises passaging the cell population emerged in step (b) at least twice. In a further embodiment, step (c) of the process comprises passaging the cell population emerged in step (b) up to four, or five times. Final harvest of the expanded population of HALPC may be conducted after P5, or optionally after P4. In another embodiment, step (c) of the process comprises passaging the cell population emerged in step (b), three or four times. In one embodiment, the cells are passaged during step (c) when at least 40 % confluence is reached. In other embodiments, a single passage in step (c) is performed when about at least 50%, 70%, or at least 90% or more confluence is reached. In a further embodiment, a passage in step (c) is performed when at least greater or equal to 80 % confluence is reached.

In yet another embodiment, the duration of step (c), wherein step (c) comprises 5 passages, is between 25 and 45 days, or between 25 and 30 days. Where step (c) comprises 2 or 3 passages, the duration of step (c) is between 10 and 24 days, or between 12 and 21 days. In yet a further embodiment of the invention, each passage of step (c) has a duration of between 5 and 8 days, preferably 6-7 days.

In a preferred embodiment, the culturing conditions of step (c) also include the use of a xeno- and serum-free culture medium comprising purified native or recombinant human serum albumin. In another embodiment, step (c) comprises the use of a xeno-and serum-free culture medium comprising purified native or recombinant human serum albumin and less than about 30 ng/mL of EGF, or less than about 10 ng/mL of EGF, or wherein said medium contains no EGF. Preferably, the xeno- and serum-free culture medium is further characterized in that it exhibits one or more of the features as described in the above embodiments for the culture media used in step (b).

Moreover, such xeno- and serum-free culture medium may be used for one or more passages of step (c). In a further preferred embodiment, the entire step (c) is conducted using only xeno- and serum-free culture media as described for step (b).

In step (c) the isolated liver progenitor cells may be plated, or seeded, onto a substrate which allows adherence of cells thereto, and cultured in a liquid culture medium as defined herein and sustaining their further proliferation.

The primary liver cells may be, for example, cultured and proliferated in step (c) in a conventional monolayer culture system, for example in the form of stacked chambers optionally comprising surface treatments or coatings which promote or allow adherence and proliferation of cells. Alternatively, and preferably, step (c) is performed in a bioreactor.

Bioreactors are cell culture systems providing a controlled microenvironment and suitable conditions which are adapted for the proliferation and expansion of cells in a reproducible, standardized manner. Compared to static or traditional mono-layer cultures, such as '2D' planar culture systems (for example T-flasks), bioreactors are scalable and can be used for larger scale or even industrial scale manufacture of cells.

Preferably, the bioreactor is used in combination with microcarriers which function as substrates and provide surface area for the adherence of the emerged liver cells of step (b) or cells undergoing passage in step (c). The microcarrier may be selected from any one of the embodiments described herein.

In one embodiment, the bioreactor is a stirred tank bioreactor. A stirred tank bioreactor may comprise an impeller system for homogenous stirring and agitation of the cell suspension during culture and expansion.

In preferred embodiments, the bioreactor is a horizontal-stirred tank bioreactor or a vertical stirred tank reactor, respectively. A horizontal stirred tank bioreactor usually has an impeller system configured to rotate about a vertical axis, for example by means of impeller blades mounted on a vertically-oriented axle or shaft, generally moving fluid, i.e. culture medium, in an upwards or downwards direction. An example of a horizontal stirred tank type of bioreactor is a spinner flask or spinner reactor, sometimes simply referred to as "spinner".

A vertical stirred tank reactor, on the other hand, is configured with a stirrer or impeller system that may be mounted on a horizontally-oriented axle or shaft such as to rotate along a horizontal axis. In one embodiment, the vertical stirred tank reactor is a vertical wheel reactor, in which the impeller system is in the form of a mixing wheel. An example of a vertical stirred tank reactor are the PBS vertical wheel reactors (PBS Biotech, Inc).

Other examples of commercially available bioreactors which may be useful or exemplary in the context of the present invention include PADReactor^{®} or PADReactor^{®} Mini, Allegro STR (Pall Corp.), ambr^{®} 250, UniVessel^{®}, Biostat STR^{®}, (Sartorius-Stedim) and Mobius^{®} STR (Merck-Millipore).

In an alternative embodiment, the bioreactor is not a stirred tank reactor, but a reactor comprising an alternative agitation means to an impeller, for example, a rocking-motion or rocking bioreactor, which is a bioreactor integrated or adapted with a rocking platform which in motion provides wave motion or turbulence to the cell culture.

The bioreactor that may be used in the present invention may comprise of at least one container, or vessel adapted for containing or holding a cell culture; a stirring or agitation means; and one or more ports, which may optionally be integrated or part of the container or vessel, and which are adapted for the input and/or output of, for example, introducing or replenishing fluids such as culture media, or gases, or for introducing microcarriers, cells, nutrient components, or adapted for harvest, or washing of cells. Preferably, all apparatus or components of the bioreactor and introduced to the bioreactor are sterile, and handled under aseptic conditions.

In one embodiment, the bioreactor is a single-use bioreactor, wherein the bioreactor is adapted to accommodate for single-use container or vessel (e.g. a bag) adapted to hold the culture composition (e.g. culture medium, microcarriers, cells, etc.).

One or more bioreactors may be used in parallel for the expansion of the cells in step (c). A bioreactor may optionally also be a system comprising, in parallel, one or more, i.e. multiple vessels or containers adapt holding the cell culture.

The volume of the bioreactor (i.e. the volume of the container or vessel adapted for holding the cell culture and in which cell expansion takes place) may optionally be in the range from about 0.1 to about 1,000 litres, or from about 0.2 to about 500 litres, or from about 0.5 liters (i.e. 500 ml) to about 200 liters, respectively. In other embodiments, the volume of the bioreactor is comprised between 0.1 and 5 liters, for example 3 liters, or 5 liters, 10 liters or 50 liters.

In one of the preferred embodiments, process step (c) involves the use of two or more different bioreactors, in particular the sequential use of two (or more) bioreactors having substantially different internal volumes. For example, the cell population emerged in step (b) may first be expanded in a bioreactor having a volume of about 0.5 L to about 10 L, and subsequently transferred or passaged to a different bioreactor having an internal volume of about 10 L to about 100 L.

A passage in step (c) may further comprise the steps of (i) seeding the cells obtained from step (b) or an earlier passage of step (c) on to microcarriers, culturing said cells under conditions to cause their expansion in a bioreactor according to any one of the embodiments described herein, and (ii) detaching and dissociating the cultured cells from the culture substrate and from each other.

As understood herein, the term `microcarrier' (or alternatively `microbead') is a particulate substrate to which cells may adhere or anchor during culture. Microcarriers are generally small, spherical particles or beads, usually with an average diameter of between 90 to 400 µm. The expression 'microcarrier', as used herein, may refer to a single bead or to a plurality of beads.

Microcarriers are added to a cell culture medium as a growth surface for adhesion-dependent cells. In particular, microcarriers provide a higher surface to volume ratio compared to monolayer cell cultures and may allow for a better process control.

Potentially suitable microcarriers may be based on synthetic polymers, such as polystyrene, polyethylene, polyvinyl alcohol (PVA), polylactide, polyglycolide, polycaprolactone, or copolymers of polylactide, polyglycolide, and polycaprolactone, such as poly(lactic-co-glycolic acid) (PLGA). In one embodiment, microcarriers based on synthetic polymers are used which are substantially non-porous.

In another embodiment, the microcarrier is based on a natural polymer, or an optionally modified biopolymer, such as a polymer comprising a polypeptide or polysaccharide. Suitable examples include, without limitation, a microcarrier based on gelatine, alginate, collagen, cross-linked polygalacturonic acid (PGA), crosslinked dextran, glycosaminoglycan, cellulose, or derivatives (such as ethers or esters) of cellulose. Optionally, the microcarrier based on an optionally modified biopolymer is porous.

In another embodiment, the microcarrier may be a digestible, or biodegradable microcarrier. A digestible microcarrier may be further digested, degraded (or dissolved), for example by use of a suitable enzyme and/or by means of a chemical agent such as a solvent, or a sequestering agent. Digestible microcarriers may be advantageous for a cell expansion process while minimizing damage or decrease to viability of the cells, and may allow for an increased viable cell harvest.

If a digestible microcarrier is used, digestion of the microcarrier is performed as a sub-step or intermediate step of step (c) of the process according to the invention, for example as part of the detachment of the expanded cells from the microcarrier before a further step of expansion and passage. The digestion of the microcarrier is also performed as part of the process of step (d) according to the invention, in order to harvest the expanded cells obtained in step (c).

In one embodiment, the microcarrier used according to the process of the present invention is a digestible microcarrier, for example a Ca²⁺ cross-linked polygalacturonic acid (PGA) polymer microcarrier, which may be digested after the harvest stage by treatment with EDTA and pectinase.

In a preferred embodiment of the process according to the invention, the microcarrier is selected from a polystyrene, gelatine, cross-linked dextran, cross-linked cellulose, polyethylene with silica, polyvinyl alcohol (PVA) and a digestible microcarrier.

The microcarrier, i.e. the surface of the microbeads that the microcarrier consists of, may be coated with a biocompatible material that facilitates the adhesion of the cells. In particular, microcarriers based on synthetic polymers preferably exhibit a coating, but also for certain biopolymer-based microcarriers a coating may be useful to further enhance the properties of the beads in the context of the process of the invention.

In one embodiment, the microcarrier is coated with a biocompatible material that facilitates the adhesion of the cells, optionally wherein the coating is a synthetic coating, synthetic polymer, biopolymer, or a chemical surface modification of the microcarrier.

As used herein, the term 'coating' or 'coated' refers to a treatment applied to a microcarrier substrate core which alters the properties of the microcarrier particle or substrate core, including, but not limited to properties such as surface charge, cell-adhesion or binding affinity, porosity, density, microcarrier diameter and other physicochemical properties. The term 'coating' as used herein may encompass not only a physical layer applied to a microcarrier substrate 'core', but also 'chemical coatings' i.e. any chemical modifications or covalent conjugations of a surface-modifying moiety to the surface of a microcarrier substrate.

Preferably said coating is a protein or polypeptide coating. The coating in particular may comprise of one or more extracellular matrix (ECM) or ECM-associated proteins or peptides. Suitable coatings include, but are not limited to collagen, preferably a human collagen; gelatin, or other peptide coating, for example, comprising of consisting of fibronectin or fibronectin peptide; vitronectin, or vitronectin peptide; a laminin or laminin peptide or any other extracellular matrix (ECM) proteins or peptide derivatives, and combinations thereof. Such coatings are preferably adapted for, and suitable for promotion of cell adhesion to the substrate.

Some microcarriers may utilize a proprietary coating. Examples for proprietary xeno-free coatings include CellBind^{®} or Synthemax II^{®} from Corning, or NunclonTM from SoloHill Eng.

In other embodiments, the microcarrier coating does not contain a polypeptide. Instead, the coating may be a synthetic or artificial coating such as a glass coating, in particular high silica glass. In other embodiments, the coating is a chemical coating or a chemical modification to the surface of the microcarrier substrate. In one embodiment, the surface modification involves the introduction of cationic trimethyl ammonium groups. In other embodiments, the surface may be modified with diethylaminoethyl (DEAE) moieties, such as in the case of DEAE-cellulose or DEAE-modified crosslinked dextran (e.g. Cytodex 1).

Dependent on the selection of the material of the microcarrier substrate and/or coating if applied, said microcarrier particles or beads may have a surface charge. In some embodiments, said microcarriers have an uncharged or neutral surface. In other embodiments, the microcarriers have a positively charged surface. In other embodiments, the microcarrier may have a slightly negatively charged surface. The charged surface may be obtained by either coating the microcarrier with a charged coating, either physically or via chemical modification or treatment of the surface core substrate.

Microcarrier substrates allowing for a neutral or uncharged surface include collagen, polystyrene, dextran, high silica glass or polyvinyl alcohol. A positively charged surface on the other hand may be obtained by utilizing a coated or treated microcarrier, for example a microcarrier coated with cationic collagen, cationic trimethyl ammonium or diethylaminoethyl.

Dependent on the selection of the material, the microcarrier may be optionally be classified as a rigid microcarrier which may include glass, or plastic (for example polystyrene microcarriers), or a soft, swellable microcarrier (for example gelatine, alginate, or dextran microcarriers).

In one embodiment of the invention, the microcarrier is coated with a surface treatment selected from peptide coating, such as collagen, gelatin, or fibronectin; a glass coating such as high-silica glass; and a chemical or surface-charge coating such as cationic trimethyl ammonium, or diethylaminoethyl.

In a preferred embodiment, the microcarrier and/or its coating which is utilized in the context of the present invention is xeno-free, i.e. free of any non-human, animal components. In particular, it is preferred that any polypeptide substrate microcarriers, or microcarrier which is modified, or coated with polypeptide or proteins is xeno-free, or is obtained via a process or culture which is free of use of any non-human, animal products or components of animal origin.

In another embodiment, the coating comprises a human recombinant peptide or protein, such as rh-collagen, rh-fibronectin, rh-vitronectin, rh-laminin, or any combinations thereof.

In one embodiment, the average diameter of a microcarrier, which is approximately spherical or is a microbead is from about 50 to 500 µm. Microcarriers may be homogenous in size.

Particularly suitable microcarriers, which are in the form of the bead or are approximately spherical, are microcarriers having a volume median diameter (d50) in the range of about 90 to 400 µm. The term volume median diameter or d50 refers to the diameter at 50% of the volume of a sample of the microcarrier. In other preferred embodiments, the microcarrier may have a volume median diameter in the range of 100 to 300 µm, or of 100 to 250 µm, respectively.

In other embodiments, microcarriers which are in the form of a bead or are approximately spherical may have a diameter expressed as the d5 value and/or the d95 value, referring to the diameter of the 5th percentile and the 95th percentile of a sample of microcarriers. For example, the d5 value may be in the range of about 50 to about 250 µm, or from about 80 to about 200 µm respectively; and/or the d95 value may be in the range of about 100 to about 400 µm, or from about 150 to about 300 µm, respectively.

In general, microcarriers may be categorized as a solid (non-porous) or a porous microcarrier. Porous microcarriers can be macroporous or microporous or a combination of macro and microporous. Macroporous carriers comprise pores of 50 nm and greater, whereas microporous microcarriers typically comprise pores of 10 to 35 µm.

Microcarriers may provide greater surface area for cellular adhesion, compared to conventional monolayer culture substrates. In one embodiment, the microcarrier on which the liver progenitor cells are seeded in step (c) for expansion have a surface area in the range of 300-6,000 cm²/g. In a particular embodiment, the microcarrier has a surface area of between 300 and 600 cm²/g. In other embodiments, the surface area of the microcarrier may be in the range of about 1,000 to about 6,000 cm²/g, or in the range of about 2,000 to about 5,000 cm²/g, respectively. In one specific embodiment, the microcarrier comprises porous beads based on an enzymatically digestible biopolymer, wherein the beads have a d50 value in the range of about 100 µm to about 250 µm and a surface area in the range from about 2,000 to about 5,000 cm²/g.

Independent of a porous or solid microcarrier, the average density of a microcarrier is usually greater than 1 g/cm³. Preferably, the density of the microcarrier is adapted for uniform and consistent suspension in the cell culture medium and for stirring or movement in the bioreactor. In one embodiment of the invention, the microcarrier used as an average density of between 1.02 and 1.12 g/cm³. In particular, the average density may be about 1.01, 1.02, 1.03, 1.04 or 1.05 g/cm³. In some cases, the average density is about 1.10, 1.11 or 1.12 g/cm³.

In one embodiment of the process according to the invention, the microcarrier is a bead having an average diameter between 90 and 400 µm, a density between 1.02 and 1.12 g/cm³ and a surface area of between 300 and 600 cm²/g.

In another embodiment, the microcarrier is a digestible microcarrier (for example, a cross-linked polygalacturonic acid polymer (PGA) microcarrier) having an average diameter of about 250 µm, a density of about 1.02-1.03 g/cm³ and a surface area of about 5000 cm²/g.

When passaged, the cultured cells are detached and dissociated from the culture substrate and from each other. Detachment and dissociation of the cells can be carried out as generally known in the art, e.g., by enzymatic treatment with proteolytic enzymes (e.g., chosen from trypsin, collagenase, e.g., type I, II, III or IV, dispase, pronase, papain, etc.), treatment with bivalent ion chelators (e.g., EDTA or EGTA) or mechanical treatment (e.g., repeated pipetting through a small bore pipette or pipette tip), or any combination of these treatments. Preferably, the cells are detached by enzymatic treatment with a proteolytic enzyme.

A suitable method of cell detachment and dispersion should ensure a desired degree of cell detachment and dispersion, while preserving a majority of cells in the culture. Preferably, the detachment and dissociation of the cultured cells would yield a substantial proportion of cells as single, viable cells, for example at least 50%, 70%, 90% of the cells or more. The remaining cells may be present in cell clusters, each containing a relatively small number of cells, for example, on average, between 1 and 100 cells.

The so detached and dissociated cells are then harvested as per step (d), or else if further expansion and passages are to be conducted under step (c), re-plated or reseeded onto a substrate which allows the adherence of cells thereto, and subsequently cultured in a medium as described above sustaining the further proliferation of HALPCs and of HALPC Progeny.

In a further embodiment, the seeding density of the cells in step (c) culture onto the microcarrier, i.e. at the first passage or subsequent passages in step (c) is at a density of between 1000-8000 cells/cm². In another embodiment the seeding density is about 5000 cells/cm², or is no more than 5000 cells/cm².

The cells may also be cultured by replating at a splitting ratio between about 1/16 and 1/2, preferably between about 1/8 and 1/2, more preferably between about 1/4 and 1/2. The splitting ratio denotes the fraction of the passaged cells that is seeded into an empty (typically a new) culture vessel of the same surface area as the vessel from which the cells were obtained.

The type of culture vessel, as well as of surface allowing cell adherence into the culture vessel and the cell culture media, can be the same as initially used and as described above, or may be different. As previously mentioned, the cells may be cultured on any appropriate support that may optionally be coated with e.g. extracellular matrix proteins (such as collagens, and preferably collagen type I) or synthetic peptides that are acceptable for clinically grade cell culturing and available in GMP grade.

Concerning the process for step (d) above relating to the final harvest and isolation of the population of HALPC, the terms "cell population" and "population of cells" refer generally to a group of cells. Unless indicated otherwise, the term refers to a cell group consisting essentially of or comprising cells as defined herein. A cell population may consist essentially of cells having a common phenotype, or may comprise at least a fraction of cells having a common phenotype. Cells are said to have a common phenotype when they are substantially similar or identical in one or more demonstrable characteristics, including but not limited to morphological appearance, the level of expression of particular cellular components or products (e.g., RNA or proteins), activity of certain biochemical pathways, proliferation capacity and/or kinetics, differentiation potential and/or response to differentiation signals or behavior during in vitro cultivation (e.g., adherence or monolayer growth). Such demonstrable characteristics may therefore define a cell population or a fraction thereof. A cell population may be "substantially homogeneous" if a substantial majority of cells have a common phenotype. A "substantially homogeneous" cell population may comprise at least 60%, e.g., at least 70%, at least 80%, at least 90%, at least 95%, or even at least 99% of cells having a common phenotype, such as the phenotype specifically referred to (e.g., the phenotype of liver progenitor or stem cells of the invention, or to progeny of liver progenitor or stem cells of the invention).

Moreover, a cell population may consist essentially of cells having a common phenotype such as the phenotype of liver progenitor or stem cells of the invention (i.e. a progeny of liver progenitor or stem cells of the invention) if any other cells present in the population do not alter or have a material effect on the overall properties of the cell population and therefore it can be defined as a cell line.

Cell count and cell viability, irrespective of the method which is applied, can be performed on the cell suspension at final harvest, subsequent to step (b) of the process, or any one of the passages of step (c), and also in the course of formulating the HALPCs or during the quality control testing. Any method known in the art can be used, such as the manual count method using a Bürker Chamber, or an automated counter, such as a NucleoCounter. The aim of these methods is to determine the total amount of cells as well as the amount of the viable ones.

The manual count method using a Burker chamber is based on the Trypan Blue exclusion test. According to one protocol for performing the analysis, the cell suspension is diluted with PBS or in any other suitable reconstitution medium, to count between 100 and 200 viable cells per chamber. Trypan blue is added to cell suspension using a ratio 1:1. The cells are counted by microscopy and cell counter. The white cells are viable cells; the blue cells are dead cells. The percentage of viable and dead cells is then calculated. Two cell counts are performed. If the difference between both counts is > 15%, a third count is carried out. As will be appreciated by a person skilled in the art, similar protocols and materials may also be used and can lead to essentially equivalent results.

Alternatively, automatic cell counters based on image cytometry may be used, such as the Nucleocounter NC-200 1-step or a similar instrument. Such image cytometer provides a high precision automatic cell counter based on fluorescent microscopy and advanced image analysis to perform image cytometric exclusion of dead cells and total cells. In the case of the Nucleocounter NC-200 1-step, a one-step method may be performed which uses Vial-Cassette^{™} containing immobilized fluorescent dyes, i.e. Acridine orange and DAPI (4',6-diamidino-2-phenylindole) that automatically stain the total and dead cell populations respectively. Again, a person skilled in the art will understand that other instruments, protocols, and materials may similarly be useful.

The cell suspension is diluted to count between 7×10⁵ and 2×10⁶ total cells/ml. The Vial-Cassette^{™} will pipette a calibrated volume and the NC-200 will automatically determine the total and dead cell concentration and the percentage of viability. All counts are performed in triplicate. The reportable value is the mean out of three valid results of three independently drawn samples. To be valid, total cell count must be between 7×10^5 and 2×10^6 total cells/ml and the coefficient of variation (CV) must not exceed 15.0% for the total cell concentration and for the viability.

Step (e) of the process for the manufacture of a population of HALPC according to the invention is establishing that the cells of the expanded cell population obtained in step (c) are indeed the desired human allogeneic liver-derived progenitor cells (HALPC). For example, this may include establishing that the cells express at least one mesenchymal marker selected from CD90, CD44, CD73, CD13, CD140b, CD29, vimentin and alpha-smooth muscle actin (ASMA), and optionally express at least one hepatic marker and/or exhibit a liver-specific activity. Alternatively, it may include establishing that the cells coexpress at least one mesenchymal marker selected from CD90, CD44, CD73, CD13, CD140b, CD29, vimentin and alpha-smooth muscle actin (ASMA) with one or more hepatic marker selected from alphafetoprotein (AFP), alpha-1 antitrypsin, HNF-4 and MRP2 transporter and optionally the hepatic marker albumin. Alternatively, it may include establishing that the cells coexpress at least one mesenchymal marker selected from CD90, CD44, CD73, CD13, CD140b, CD29, vimentin and alpha-smooth muscle actin (ASMA) with hepatocyte growth factor (HGF) and optionally also prostaglandin E2 (PGE2).

In a further preferred embodiment, step (e) comprises establishing that the HALPCs obtained in step (c) or (d) are positive for CD90, CD73, vimentin and ASMA. Further alternative marker and/or activity profiles to be established in step (e) have been described above.

As used herein, the term "establish" or "establishing" should be interpreted broadly and in the context of controlling a pharmaceutical manufacturing process. While it is necessary to verify the identity of the cells produced by a batch process, it may not be necessary or feasible to test the entire range of cell characteristics for every batch, such as the entire marker or activity profile of the cells. It may be sufficient to check only some of the key markers or cell properties that have been selected as indicative for a specific cell type or subtype.

Said step of establishing the identity of the cells, e.g. by verifying the expression of markers may be performed after any passage of step (c) for example after the first passage (P1), second passage, (P2), etc, for example for quality control purposes.

The step of establishing the identity of the cells, e.g. by verifying the expression of certain markers may also be performed subsequent to the final passage of step (c), for example on the harvested population of cells obtained as per step (d) of the process. In other words, step (e) may be performed before or after step (d), or in parallel with step (c).

In addition, establishing the identity of the cells, e.g. by checking the expression of selected markers, may optionally also be performed on a sample of emerged cell population obtained from culturing step (b). However, such additional testing of the cells obtained in the earlier process step (b) cannot replace step (e).

Again, potentially useful markers for establishing the identity of the cells may be selected from the preferred or optional markers disclosed herein, and those skilled in the art will appreciate that for routine identity checks it may not be necessary or useful to check the entire marker profile of the cell. Normally it will suffice to verify the presence and/or absence of a few markers that are specific selected as pivotal for establishing the identity of the cells produced in a batch process.

In a further embodiment, the HALPCs prepared according to the invention are
a. positive for α-smooth muscle actin (ASMA), CD140b and optionally albumin (ALB); and
b. negative for Cytokeratin-19 (CK-19).

In one of the particularly preferred embodiments, the HALPCs prepared according to the invention are positive for CD90, CD73, vimentin and ASMA, and negative for CK-19.

In a further embodiment, the HALPCs prepared according to the invention are
a. positive for α-smooth muscle actin (ASMA), CD140b and optionally albumin (ALB) and Sushi domain containing protein 2 (SUSD2); and
b. negative for Cytokeratin-19 (CK-19).

In a further embodiment, the cells may also be phenotyped in terms of being
a. positive for α-smooth muscle actin (ASMA), CD140b and optionally albumin (ALB);
b. negative for Sushi domain containing protein 2 (SUSD2) and Cytokeratin-19 (CK-19).

In a further embodiment, the cells may also be phenotyped or measured as positive to
a. At least one hepatic marker selected from HNF-3B, HNF-4, CYP1A2, CYP2C9, CYP2E1 and CYP3A4 and optionally albumin;
b. At least one mesenchymal marker selected from Vimentin, CD90, CD73, CD44, and CD29;
c. At least one liver-specific activity selected from urea secretion, bilirubin conjugation, alpha-1-antitrypsin secretion, and CYP3A4 activity;
d. At least one marker selected from ATP2B4, ITGA3, TFRC, SLC3A2, CD59, ITGB5, CD151, ICAM1, ANPEP, CD46, and CD81; and
e. At least one marker selected from MMP1, ITGA11, FMOD, KCND2, CCL11, ASPN, KCNK2, and HMCN1.

In a further optional embodiment, said cells are negative for HLA-DR.

In a further embodiment, the HALPCs are further tested and established as being negative for the markers CD133, CD45, CK19 and/or CD31.

Further features can be also determined for HALPCs of the above embodiment in any functional and technical combination, for instance by measuring positivity for at least one further marker selected from ATP2B4, ITGA3, TFRC, SLC3A2, CD59, ITGB5, CD151, ICAM1, ANPEP, CD46, and CD81. In some of such embodiments, HALPCs can be measured negative for at least one further marker selected from the group consisting of ITGAM, ITGAX, IL1R2, CDH5, and NCAM1. In some of such embodiments, HALPCs can be measured negative for at least one of HP, CP, RBP4, APOB, LBP, ORM1, CD24, CPM, and APOC1.

A master cell stock (MCS) as used herein may be defined as a population of emerged cell population obtained from culturing step (b) which have been expanded according to step (c) to a given number of cells, which may be aliquoted and stored for use as a starting material for the production of further, but limited lots of the final cell product, i.e. in context of the present invention, the HALPCs.

The primary liver cell population obtainable from step (b) of the process according to the present invention, i.e. culturing primary liver cells from the liver cell suspension in the xeno-free and serum-free medium, in accordance with any one of the embodiments described herein, or alternatively the cell population harvested from any passage of step (c), may be aseptically formulated, filled into vials and cryo-stored as a master cell stock. Said master cell stock may be later thawed, and seeded into appropriate culturing vessel or bioreactor for continuance of the process, which may comprise further steps (c), (d), and (e).

In one embodiment, the process according to the invention further comprises the steps of:
(c1) harvesting the cell population obtained in step (b) or after any passage of step (c);
(c2) adding a cryoprotectant to the cell population harvested in step (c1) to obtain a cryoprotected cell formulation;
(c3) freezing said cryoprotected cell formulation; and
(c4) thawing said frozen cryoprotected cell formulation;
wherein steps (c1) to (c4) are performed as sub-steps of step (c).

In one embodiment, steps (c1) to (c4) are performed as a sequence of sub-steps of step (c) immediately at the beginning of step (c). Accordingly, in step (c1), the cell population obtained in step (b) is harvested. The subsequent steps remain the same:
(c2) adding a cryoprotectant to the cell population harvested in step (c1) to obtain a cryoprotected cell formulation; (c3) freezing said cryoprotected cell formulation; and
(c4) thawing said frozen cryoprotected cell formulation.

In another embodiment, steps (c1) to (c4) are performed as a sequence of sub-steps of step (c) following one (i.e. the first) passage of the cell population in accordance with step (c). In other words, step (c) may be conducted as P1 followed by the generation of the MCS (i.e. steps (c1) to (c4)), followed by P2 (optionally followed by P3, which is optionally followed by P4). Alternatively, step (c) may be conducted as P1 followed P2, followed by the generation of the MCS (i.e. steps (c1) to (c4)), followed by P3 (optionally followed by P4).

Step (c4) comprising the thawing said frozen cryoprotected cell formulation may be conducted when further production of a population of HALPCs is required or planned. The time period which may elapse between step (c3) and step (c4) may be up to 12 months, or up to 24 months, or even longer than 24 months, respectively.

In a further aspect, the invention relates to a cryoprotected cell formulation obtained in step (c2) or step (c3) of said process.

As used herein, a cryoprotectant is an agent which is capable of protecting living cells from freezing damage. Examples of potentially useful cryoprotectants include dimethyl sulfoxide (DMSO), glycols such as glycerol, sugars and sugar alcohols.

In one embodiment, the cryoprotectant is DMSO. In this case, preferably, the content of DMSO in the cryoprotected cell formulation is not higher than about 5%. In other embodiments, the cryoprotected cell formulation comprises no more than 2% DMSO. In an alternative embodiment, the cryoprotectant comprises no DMSO.

In another embodiment, the concentration of the cells in the cryoprotected cell formulation is about 10×10⁶ cells per vial, or between 2 to 20 million cells per vial (e.g. for a vial with a volume of 6 ml). Said amount of cells may be determined by the above defined methods or methods known in the art for counting cells.

In another aspect, the invention relates to the use of a xeno- and serum-free culture medium comprising purified native or recombinant human serum albumin for culturing primary liver cells obtained or obtainable from a human liver such as to produce human allogeneic liver-derived cells (HALPC). In particular, the invention provides the use of a xeno- and serum-free culture medium comprising purified native or recombinant human serum albumin which is further characterized in that it contains less than 30 ng/mL of epidermal growth factor (EGF) for the manufacture of human allogeneic liver-derived cells. In related embodiments, the xeno- and serum-free culture medium exhibits one or more further features as described above in the context of the disclosure of the process steps (b) and/or (c). In a further aspect, the invention relates to human allogeneic liver-derived progenitor cells, such as an isolated HALPC, obtainable by the process of the invention, as well as an isolated population of human allogeneic liver-derived progenitor cells obtainable by the process of described herein, and according to any one or combination of the related embodiments described herein.

The inventors have discovered that HALPCs prepared according to the invention show certain minor but potentially advantageous differences to the cells previously obtained by a process relying on culture media comprising foetal bovine serum. In one aspect, the HALPCs prepared according to the invention have the same morphology, but a slightly smaller size. For example, the mean cell diameter is frequently about 5 to 20%, such as 10 to 15%, smaller for cells prepared according to the invention, compared to otherwise similar cells prepared with foetal bovine serum. For example, in one comparison, the mean cell diameter for HALPCs prepared according to the invention was in the range of approx. 14 µm, whereas similar HALPCs prepared with serum had a mean diameter of approx. 16 µm, as determined using an automatic cell counter based on image cytometry (such as a NucleoCounter NC-200). In general, a smaller cell size is potentially beneficial for handling and administering the cells as a smaller diameter can translate into slower sedimentation and better syringability.

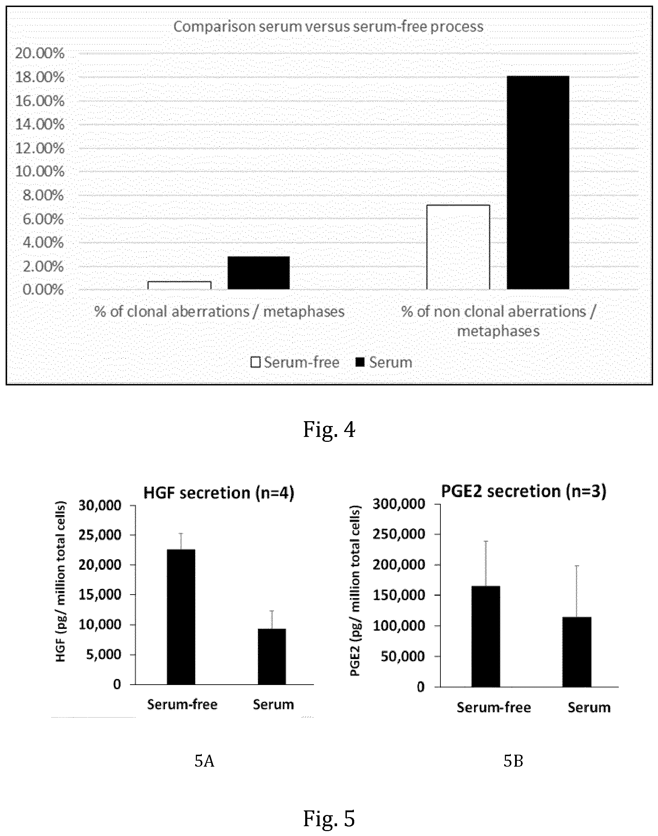

Moreover, the HALPCs prepared according to the invention show a decreased average level of clonal as well as non-clonal aberrations per metaphase compared to HALPCs prepared with foetal bovine serum, as described in Example 5 and shown in Figure 4. In general, a low incidence of aberrations is desirable in the context of cell therapy.

In yet a further aspect, the invention provides human allogeneic liver-derived progenitor cells that are positive for CD90, CD73, vimentin and ASMA, and that exhibit in average less than about 2.5% clonal aberrations per metaphase and/or less than about 15% non-clonal aberrations per metaphase.

The invention further relates to said isolated population of human allogeneic liver-derived progenitor cells for use in the treatment of a liver disease, or the use of said isolated population of human allogeneic liver-derived progenitor cells in the manufacture of a medicament for the treatment of a liver disease. Moreover, the invention may also encompass a method of treating a liver disease, said method comprising a step of administering to a patient in need thereof said isolated population of human allogeneic liver-derived progenitor cells. For example, the cells may be used for treating, acute-on-chronic liver failure (ACLF), or for treating a non-alcoholic fatty liver disease, such as non-alcoholic steatohepatitis (NASH).

The following examples serve to illustrate the invention, however should not to be understood as restricting the scope of the invention.

### EXAMPLES

### EXAMPLE 1 -Manufacture of HALPC Cells in xeno-free and serum-free media

### HALPCs were prepared according to the following general procedure:

### Liver cell suspension (LCS) preparation

Liver cell suspensions were produced from donated livers. The liver cells were obtained using the technique of multi-step collagenase perfusion at 37°C. The whole liver or the segment is weighted. Cannulae were inserted into the accessible vasculature and connected with the perfusion pump(s). The major steps in the production of LCS comprise the loosening of desmosomes, the liver digestion, the liver disruption, the filtration, the liver cell separation by centrifugation and the cryopreservation of the final LCS.

The liver tissue was perfused with a calcium complexation medium, containing a Ca²⁺ chelating agent as well as antibiotics, in order to remove the residual blood and to loosen cell junctions (desmosomes) by complexing Ca²⁺. A digestion medium containing collagenases +/- protease was then infused into the liver, rapidly breaking down the collagen matrix. When it becomes fragile, the liver was placed on the sieves. After stopping the collagenase buffer flow, the cannula was removed and the liver tissue was mechanically disrupted and lacerated. The cells were released from the digested tissue by gentle shaking, and then filtered and rinsed through metal sieves or through a nylon mesh in order to remove undigested liver pieces (e.g. Glisson's liver capsule, connective tissue, vascular tree, and non-perfused/undigested liver tissue portions). The suspension was then collected for centrifugation. The liver cells were concentrated once and washed 2 times by centrifugation for a total of 3 spins in a cold wash medium and then concentrated before freezing and storing. Before freezing, the supernatant was discarded and the liver cells pellet was resuspended in a freezing medium, with or without preliminary addition of a buffer and sampling for cell viability, and stored in bags or vials at ≤130 °C (at a cell concentration of between 7.5- 20×10⁶ cells/mL in. Optionally, freshly prepared LCS may be used and directly processed for manufacturing of the HALPCs.

### Emergence (P0)

To prepare the HALPCs, a bag of cryopreserved liver cell suspension (± 650M cells) was thawed in reconstitution medium (sodium bicarbonate, heparin, glucose, human albumin) and centrifuged twice for 10 minutes ((+4 °C) at 224g). After centrifugation, the cell pellet was resuspended in an adequate volume of the selected culture medium and counted using NucleoCounter NC-200 (Chemometec).

Sterile culture flasks (T-flasks with CellBIND surface) were seeded at 66666/cm² or alternatively 20,000/cm², at a surface-to-volume ratio of 0.20 mL/cm² or 0.16 mL/cm².

The cultures were maintained at +37°C in humidified atmosphere containing 5% COz. The medium was replaced twice a week (e.g. every 3 to 4 days). When the culture was near confluence (≥80%), cells were rinsed once with DPBS (Dulbecco's phosphate buffered saline) at a surface-to-volume ratio of 0.20 mL/cm² or 0.16 mL/cm², and then detached by treatment with TrypLE^{™} Select Enzyme 1x at a surface-to-volume ratio of 0.04 mL/cm². This latter was quenched with medium (DMEM (Dulbecco's Modified Eagle Medium)/2% HSA) at a surface-to-volume ratio of 0.12 mL/cm² or 0.08 mL/cm². The cell suspension was harvested in a conical sterile tube and centrifuged at 224g for 10 minutes (+15-25 °C). The supernatant was then discarded, and the cell pellet resuspended with culture medium. Samples were taken for counting cells via the NucleoCounter NC-200 device (Chemometec).

### Expansion (P1 to P5)

P1 (Passage 1) - Cells from P0 were replated or reseeded at a 5000 cell/cm² density in sterile culture vessels in the selected culture medium. The cultures were maintained at +37°C in humidified atmosphere containing 5% COz. The medium was replaced twice a week (e.g. every 3 to 4 days), until near confluence (≥ 80%) was reached. Subsequent processing steps to harvest and detach the cells from the substrate/vessel as above for emergence P0 was repeated for further passage i.e. sub-cultivation. As understood herein the passage number (P1 to P5) simply refers to the number of times the cells in the culture are subcultured.

P2 (Passage 2) to P5 (Passage 5) - The above steps as for P1 were repeated, up to a final P5 (Passage 5) upon which a final harvest of the HALPC cells (P5/H) was conducted. All cells were pooled to form the final harvest.

Subsequent to harvest (and as could be done optionally at any one or combination of the P1 to P5 passages), the cells were sampled and analyzed, for example for cell viability, purity and identity(e.g. for the expression of CD90, CD73, vimentin and/or ASMA) .

At harvest, the HALPC cells were resuspended in a cryopreservation solution (CryoStore CS5) and cryopreserved.

The culture media used for emergence of the HALPC (P0) and for the expansion steps (P1 to P5) were selected from serum-free and xeno-free media (medium A, medium B and medium C, respectively). All serum-free and xeno-free media comprised a basic mixture of inorganic metal salts, nutrients such as amino acids, and vitamins, and had a pH in the range from pH 7.0 to 7.4, such as known from the composition of Dulbecco's Modified Eagle Medium (DMEM) or DMEM/F-12. The media also contained glutamine or glutamate, or a precursor thereof such as L-alanyl-L-glutamine, and glucose at a level of not more than 2 wt.-%, and one or more fatty acids.

Medium A further comprised purified native human proteins and also recombinant human protein components, including purified human plasma-derived serum albumin and transferrin, and recombinant human insulin. The medium also comprised a human growth factor, but no EGF. A commercially available product of this type is PRIME-XV^{®} MSC Expansion XSFM (FUJIFILM Irvine Scientific), which was used in this Example 1.

Medium B also comprised native purified human serum albumin and recombinant human insulin. In this Example, the commercial product, StemMACSOO MSC Expansion kit XF (Miltenyi Biotech) was used as medium B.

Medium C was a tailor-made culture medium composition primarily characterised in that it contained a recombinant human serum albumin derived from rice plants (e.g. Optibumin^{®} and/or Cellastim^{®}, both available from InVitria), of which at least some may be associated with lipids, in particular fatty acids or fatty acid compounds similar to those typically associated with the albumin in human serum. It also contained recombinant human transferrin (available as Optiferrin^{®}, InVitria). The growth factor EGF was absent.

For comparison, culture media containing animal-derived serum was also used as reference for producing the HALPC cells: DMEM supplemented with 9.1% FBS (fetal bovine serum); William's E supplemented with 9.1 % FBS (fetal bovine serum), insulin and dexamethasone. The plating density used for the Williams E reference at P0 was only at 66666/cm².

### Results

In the processes conducted with the xeno- and serum-free media, it was observed, compared to the reference media comprising FBS, that the duration of emergence of HALPC cells (P0) was significantly shorter (Table 1).

**Table 1 Emergence Duration**

| | Willia ms-E | DMEM/ 9.1% FBS | Medium B | | Medium A | | Medium C | |
|---|---|---|---|---|---|---|---|---|
| Days | 66k (n= 17) | 66K (n=7) | 20K (n=7) | 66K (n=6) | 20K (n=12) | 66K (n = 3) | 20K (n= 3) | 66K (n=5) |
| Min. | 15 | 9 | 7 | 6 | 8 | 7 | 11 | 8 |
| Median | 19 | 22 | 8 | 7 | 11 | 8 | 16 | 11 |
| Max. | 23 | 34 | 14 | 10 | 21 | 11 | 17 | 14 |
| Mean | 19.29 | 20.14 | 8.714 | 7.667 | 8.667 | 8.667 | 14.67 | 10.8 |
| Std Dev. | 2.085 | 9.856 | 2.563 | 1.506 | 3.742 | 2.082 | 3.215 | 2.387 |
| Coeff. Var. (%) | 10.80 | 48.93 | 29.42 | 19.64 | 31.18 | 24.02 | 21.92 | 22.11 |

Moreover, the duration of culture in the expansion steps, i.e. time from passage P1 to achive P5/harvest was also significantly shorter (Table 2).

**Table 2 Culture Duration (Time to achieve P5 Harvest)**

| | Willia ms-E | DMEM/ 9.1% FBS | Medium B | | Medium A | | Medium C | |
|---|---|---|---|---|---|---|---|---|
| Days | 66k (n= 17) | 66K (n=7) | 20K (n=7) | 66K (n=6) | 20K (n=12) | 66K (n = 3) | 20K (n= 3) | 66K (n=5) |
| Min. | 46 | 39 | 24 | 24 | 25 | 24 | 35 | 26 |
| Median | 54 | 53 | 25 | 25 | 29.5 | 29 | 36 | 28 |
| Max. | 64 | 94 | 30 | 27 | 38 | 29 | 55 | 42 |
| Mean | 54.88 | 53.29 | 25.86 | 25.33 | 29.92 | 27.33 | 42 | 30.8 |
| Std Dev. | 6.48 | 19.55 | 2.116 | 1.366 | 4.358 | 2.887 | 11.27 | 6.535 |
| Coeff. Var. (%) | 11.81 | 34.74 | 8.18 | 5.39 | 14.57 | 10.56 | 26.83 | 21.22 |

It was also determined that a higher frequency of population doublings per passage is achieved for the cultures conducted in the xeno- and serum-free medium, or in other words, a higher yield of cells. This is reflected by higher values of cumulative population doubling level (cPDL), which refers refers to the total number of times the cells in the population have doubled since their first expansion (from P1) after their primary isolation in vitro, and is also a measure of the 'age' of the particular culture (Table 3).

**Table 3 Culture performance (cPDL starting from P1)**

| | Willia ms-E | DMEM/ 9.1% FBS | Medium B | | Medium A | | Medium C | |
|---|---|---|---|---|---|---|---|---|
| cPDL | 66k (n= 16) | 66K (n=6) | 20K (n=7) | 66K (n=6) | 20K (n=11) | 66K (n = 3) | 20K (n= 3) | 66K (n=5) |
| Min. | 7 | 7 | 14 | 15 | 12.96 | 16 | 9 | 12 |
| Median | 11 | 12 | 17 | 16 | 15 | 16 | 13 | 14.53 |
| Max. | 15 | 15 | 19 | 19 | 18 | 17 | 16 | 18 |
| Mean | 10.85 | 11.33 | 16.57 | 16.17 | 15.65 | 16.33 | 12.67 | 14.77 |
| Std Dev. | 2.494 | 2.944 | 1.988 | 1.472 | 1.733 | 0.5774 | 3.512 | 2.69 |

Functionality such as immunomodulatory activity and secretome analysis (e.g. Hepatocyte growth factor, or HGF secretion levels), and cell viability was tested and was found to be generally comparable between the liver progenitor cells obtained from the serum-free as well as the serum-containing cultures. Identity and purity testing of the HALPC cells produced from the xeno- and serum-free media, was also generally found to be comparable, and met acceptance criteria established for the HALPCs. Moreover, the safety profiling including karyotypic stability, as well as senescence and tumorgenicity for samples of the cells obtained from the xeno- and serum-free media cultures were found to fully conform with standard requirements and acceptance criteria, and were comparable with values obtained for cultures based on the reference media or better.

Interestingly, it was also observed in the process comparisons that the yield for the cells produced from the xeno-free and serum-free culture media productions was approximately similar at the lower cell plating density of 20,000 cells/cm² as for the higher density of 66,666 cells/cm². Unexpectedly, it was observed that the liver cells generally successfully emerged at P0 across all the xeno-free and serum-free culture media processes at the lower density plating, where in comparison emergence failed at this plating density for the control DMEM/9.1% FBS medium (Table 4).

The lower cell density supported by the xeno-free and serum free culture medium advantageously provided for a more efficient use of the liver cell suspension stock during production.

**Table 4 - Emergence**

| | Williams-E | DMEM/ 9.1% FBS | | Medium A | | Medium B | | Medium C | |
|---|---|---|---|---|---|---|---|---|---|
| LCS Batch | 66k | 20K | 66K | 20K | 66K | 20K | 66K | 20K | 66K |
| 1 | ✔ | ✔ | ✔ | ✔ | ✔ | ✔ | ✔ | ND | ND |
| 2 | ✔ | X | ✔ | ✔ | X | ✔ | ✔ | ND | ND |
| 3 | ✔ | X | ✔ | ✔ | ✔ | ✔ | ✔ | ND | ND |
| 4 | ✔ | X | ✔ | ✔ | X | ✔ | ✔ | ✔ | ✔ |
| 5 | ✔ | X | ✔ | ✔ | X | ✔ | ✔ | ✔ | ✔ |
| 6 | ✔ | X | ✔ | ✔ | ✔ | ✔ | ✔ | ✔ | ✔ |
| 7 | ✔ | ND | ND | ✔ | ND | ND | ND | ND | ✔ |
| 8 | ✔ | ND | ND | ✔ | ND | ND | ND | ND | ✔ |
| 9 | ✔ | ND | ND | ✔ | ND | ND | ND | ND | ND |
| 10 | ✔ | ND | ND | ✔ | ND | ND | ND | ND | ND |
| 11 | X | ND | ND | ✔ | ND | ND | ND | ND | ND |

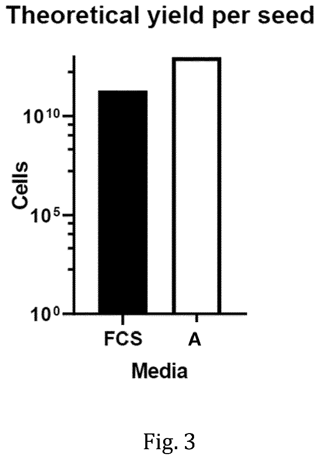

As depicted in Figure 2, which is a graphical comparison of the overall culture time from emergence P0 up to expansion passage 5 for a production run conducted with one of the tested xeno-and serum-free culture medium A (data points depicted as unfilled circles) and the serum-culture medium containing FCS (data points depicted as filled circles), the overall culture time to P5 for the serum culture medium process is higher at all passages. A total culture time of 48 days is observed for the production run conducted in the serum-containing reference culture medium, compared to a total time of 29 days for production in serum-free and xeno-free medium A. This gives a theoretical output yield per seed which is calculated to be almost 50 times higher for process conducted in the xeno-free and serum-free culture medium compared to the reference culture media (Figure 3).

### EXAMPLE 2 - Preparation of a Master Cell Stock (MCS) of HALPCs.

A master cell stock of HALPCs was prepared subsequent to passage 1 (P1) of Example 1 from one of the cultures based on a xeno-free and serum-free medium. At P1, the cells were harvested when ca. 80% confluence was observed, and the resulting cell pellet was resuspended in a cryopreservation medium (Cryostor-5). The stock was created by freezing vials filled with about 10-50 million viable cells per vial. The vials were filled under aseptic conditions.

Said stock may be used in further manufacturing runs (for example, up to 25 runs) to provide HALPC cells, as opposed to only a single production directly from liver cell suspension. The faster and also more efficient emergence (P0), as noted in Example 1, which led to increased yield and faster production times of cells as observed in the use of the xeno- and serum-free media provided the opportunity to create the master cell stock.

### EXAMPLE 3 - Bioreactor Expansion of HALPCs on microcarriers from the MCS.

The following is a representative general procedure for the expansion of HALPC from a master cell stock (MCS) prepared according to the protocol of Example 2.

One or more vials from the MCS are thawed and cells are seeded onto microcarriers in a small bioreactor (for example, 100 mL or 3L bioreactor such as a PBS vertical-wheel bioreactor) in a culture medium, such as a xeno-free and serum-free medium as used in Example 1 (such as Medium A). After the attachment phase (static or semi-static) of the cells to the microcarrier, the microcarriers are stirred and the volume of the medium in the bioreactor adjusted. The microcarriers are seeded with sufficient cells in order to obtain, on average, at least one cell per microcarrier.

The agitation speed in the bioreactor is adjusted to control and minimize sheer forces but at a sufficient rate to avoid sedimentation of the microcarriers at the bottom of the vessel and to avoid clumping. Growth is monitored by measuring metabolic parameters such as glucose, lactate, glutamine and ammonia. Media is refreshed ± on day 4, harvest is ± on day 6.

In a typical process, the concentration of microcarriers is provided at 15g of microcarriers per litre. For example, a microcarrier with an approximate surface area of 360 cm²/g would provide ca. 5400 cm² of culture surface. About 15g of microcarriers can provide ± 45M microcarriers so with seeding at approximately 1 cell per microcarrier, the amount of cells in the culture should be in the range of at least about 45-50M cells per litre.

At optimization, a 20-fold expansion can be expected within 6-7 days, generating up to ±1B cells per litre.

At the end of the culture, the media are drained and the microcarriers are rinsed with buffer before incubating them with TrypLE under low agitation. The cells are separated from the microcarriers using a sieve-in-a-bag system, i.e. the cells pass through the sieve (usually 70µm) and the larger microcarriers are retained. Alternatively, in the case of digestible microcarriers, the microcarriers are incubated with a digestion agent (such as pectinase for crosslinked dextran microcarriers) and the resulting mixture is washed to reveal the cells. In both cases, the cell suspension is then run through a centrifugal system (kSep^{®}) for washing and concentration. The loss of cells during downstream processing is expected to be around 15%.

It was found that this expansion step can be reproduced and/or repeated in a bioreactor of a size up to 50 L one or two times (passage P2, passage P3). In this case, an overall production run typically takes roughly 14 days.

### EXAMPLE 4 - Bioreactor expansion of HALPC-seeded microcarriers

The following study evaluated HALPC expansion using microcarriers and a bioreactor. The study was performed according to the general culturing and harvest procedure outlined in Example 3, except that the cells were obtained and used either as fresh cells or thawed cells from different passages.

In particular, expansion was tested on microcarriers in a vertical-wheel stirred bioreactor system (PBS Biotech), in spinner vessels as an example for a horizontal stirred tank bioreactor. T-Flasks (T175 CellBIND^{®}, Corning^{®}) were used as a control. The microcarrier substrates tested included collagen-coated microcarriers (Solohill Eng.), and dissolvable Synthemax^{®} II microcarriers (Corning^{®}).

Before use, the microcarriers were hydrated and reconstituted in accordance instructions. Cells were seeded at 5000 cells/cm² in the selected microcarriers culture vessels. The culture medium used was DMEM/9% FBS medium.

In a test using collagen-coated microcarriers (provided at 15 cm²/mL, and at 5000 cells/cm² seeding density) and DMEM/9% FBS culture medium, it was observed that in both bioreactor/microcarrier systems (vertical wheel and spinner) provided at day 7 higher number of viable cells per cm² compared to the control (Figure 1). Similar results were observed at day 7 in the test using the dissolvable Synthemax^{®} peptide surface-treated microcarriers (provided at 5 cm²/mL, and at 5000 cells/cm² seeding density) and DMEM/9% FBS culture medium (Figure 2).

### EXAMPLE 5 - Karyotype analysis: Comparison of HALPCs prepared according to invention with conventionally prepared HALPCs

A series of batches of HALPCs were prepared either according to the invention, following the procedure of Example 1 and using Medium A, and compared to batches that were produced with essentially the same process except that in the comparative batches the culture medium used in steps (b) and (c), i.e. both during emergence and expansion, was a conventional medium comprising fetal bovine serum.

Karyotype analysis was performed on cell samples obtained from these batches as follows:
Freshly collected cells were seeded in 3x T175 CellBind at a density of 5,000 viable cells/cm² in the usual culture medium. The cells were then maintained in culture until confluence of around 70% (the confluence must be between 30 and 80%).

Blocking the cells in metaphase of mitosis: The cells were then incubated with a solution of N-deacetyl-N-methyl-colchicine at 0.1 ug/ml of medium for 18 hours (+/-2 hours) at +37°C to stop polymerization of the tubulin present in the microtubules of the cells and so prevent the formation of the mitotic spindle, which blocks the cells at metaphase stage.

Collection of cells: Next, the cells were collected using trypsination followed by centrifugation at 1,200 rpm and room temperature.

Hypotonic shock: Dilute potassium chloride solution of +37 °C was added dropwise to the cells so that the cell pellets slowly disaggregated and achieved a homogeneous suspension. Subsequently, the cells were incubated for 25 minutes at +37°C to allow the KCI to induce a hypotonic shock in the cells making them swell through the osmotic pressure difference. This swelling allows the spreading of the cells and the analysis of the chromosomes.

Fixation of cells: Subsequently, the cells were fixed at room temperature using a mixture of acetic acid and methanol (3:1) which was added slowly to the cells, followed by incubation at room temperature and centrifugation at 1,200 rpm at room temperature. Several further cycles of resuspending the pellet in the fixing mixture, incubation and centrifugation at 1,200 rpm at room temperature were performed.

Spreading and staining: The fixated samples were spread on slides. After drying, samples were denatured with NaH2PO4H2O solution, washed with water and stained with a 4% Giemsa solution (G-banding). After staining, the slides were washed again with water and air-dried.

Chromosome analysis: The metaphases were counted, photographed and analyzed to establish a karyotype profile. A minimum of 20 metaphases was analyzed.

Results: It was found that the number of both clonal and non-clonal chromosomal aberrations was substantially lower for HALPCs produces in batches that were processed according to the invention compared to conventionally manufactured HALPCs, as shown in Figure 4. More specifically, the mean occurrence of clonal aberrations was 0.71% for HALPCs prepared according to the invention, compared to 2.86% for conventionally prepared HALPCs; the occurrence of non-clonal aberrations was 7.14% for HALPCs prepared according to the invention, compared to 18.09% for conventionally manufactured HALPCs.These values were obtained from 7 batches for each process and over 20 metaphases.

In conclusion, the present invention not only allows for an increased production efficiency of HALPCs, but also leads to improved cells that show a higher chromosomal stability than conventionally produced HALPCs.

### EXAMPLE 6 - HGF and PGE2 secretions

A series of batches of HALPCs were prepared either according to the invention, following the procedure of Example 1 and using Medium A, and compared to batches that were produced with essentially the same process except that in the comparative batches the culture medium used in steps (b) and (c), i.e. both during emergence and expansion, was a conventional medium comprising fetal bovine serum. Subsequently, the secretion of HGF and PGE2 by the cells was determined.

The secretion of the anti-fibrotic factors HGF by HALPC was measured as follows. HALPC were seeded in a T75 or T25 flask at 30.000 viable cells/cm², with the first 48h in DMEM/9%FBS and the last 24h incubation in DMEM/0.5% FBS. The FBS concentration was reduced to 0.5% for the last 24 hours to avoid any interference of this component with the measurement by ELISA. After the incubation, culture medium was harvested, centrifuged, aliquoted and frozen at -80°C. In parallel, cells were trypsinized and counted in order to report the quantity of HGF secreted by cells in the culture medium to the cell quantity (pg HGF/10⁶ cells). HGF concentration in cell culture medium was assessed using a specific Quantikine ELISA (Bio-Techne, R&D Systems brand, #DHG00). As shown in Figure 5A, the secretion of HGF was increased in batches prepared in Medium A (serum- and xeno-free).

| Medium | Mean HGF secretion (pg/10⁶ total cells) |
|---|---|
| Medium A | 22,628.75 |
| DMEM | 9,350.85 |

The secretion of PGE2 by the HALPCs was measured in the presence of a mixture of pro-inflammatory cytokines IL-1β, IFN-γ and TNF-α using ELISA, that means under pro-inflammatory conditions. HALPC were seeded in a T75 or T25 flask at 30,000 cells/cm² in DMEM/9% FBS. After 24h incubation, the cell culture medium was replaced by a pro-inflammatory medium (DMEM/9.1% FBS + TNFα, IL-1β and IFNy). After 24h incubation, the proinflammatory medium was removed and the cells were incubated in fresh DMEM/0.5% FBS. After 24h incubation, culture medium was harvested, centrifuged, aliquoted and frozen at -80°C. PGE2 concentration in cell culture medium were assessed using a specific competitive ELISA kit (Prostaglandin E2 ELISA kit, Abeam, #ab133021). In parallel, cells were trypsinized and counted using NC-200 one-step method in order to report the quantity of PGE2 secreted by total cells in the culture medium to the cell quantity (pg HGF/10⁶ cells). As shown in Figure 5B, the secretion of PGE2 under pro-inflammatory conditions is increased in batches prepared in Medium A (serum- and xeno-free) compared to conventionally prepared batches.

| Medium | Mean PGE2 secretion (pg/10⁶ total cells) |
|---|---|
| Medium A | 165,369.18 |
| DMEM | 114,923.40 |

## Claims

1. An in vitro process for the manufacture of a population of human allogeneic liver-derived progenitor cells (HALPC), comprising the steps of:
(a) providing a suspension of primary liver cells obtained from a human liver;
(b) culturing the primary liver cells comprised in said suspension until the emergence of a population of cells having an elongated shape and mesenchymal morphology;
(c) culturing the cell population emerged in step (b) under conditions that cause its expansion;
(d) harvesting the expanded cell population obtained in step (c);
wherein said cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and optionally express at least one hepatic marker and/or exhibit a liver-specific activity; and
wherein culturing conditions of step (b) include the use of a xeno- and serum-free culture medium comprising purified native or recombinant human serum albumin.

2. The process of claim 1, wherein the xeno- and serum-free culture medium is further **characterized in that** it contains less than 30 ng/mL of epidermal growth factor (EGF).

3. The process of claim 1 or claim 2, further comprising a step of
(e) establishing that said cells of the expanded cell population obtained in step (c) are human allogeneic liver-derived progenitor cells (HALPC) that express at least one mesenchymal marker selected from CD90, CD44, CD73, CD13, CD140b, CD29, vimentin and alpha-smooth muscle actin (ASMA), and optionally express at least one hepatic marker and/or exhibit a liver-specific activity.

4. The process of any one of the preceding claims, wherein the human allogeneic liver-derived progenitor cells obtained in step (c) or (d) are positive for CD90, CD73, vimentin and ASMA.

5. The process of any one of the preceding claims, wherein the xeno- and serum-free culture medium comprises lipids, preferably selected from one or more fatty acids, and wherein the lipids are associated with or bound to the purified native or recombinant human serum albumin.

6. The process of any one of the preceding claims, wherein the xeno- and serum-free culture medium comprises:
(i) a purified native or recombinant human transferrin, and wherein the human transferrin has optionally been pre-treated with an iron compound;
(ii) recombinant human insulin; and
(iii) a selenium compound, such as sodium selenite.

7. The process of any one of the preceding claims, wherein the xeno- and serum-free culture medium comprises basic fibroblast growth factor (FGF2) at a level of 30 pg/mL or higher, and optionally acidic fibroblast growth factor (FGF1) at a level of 5 pg/mL or higher.

8. The process of any one of the preceding claims, wherein the xeno- and serum-free culture medium comprises human fibronectin and/or human vitronectin, such as recombinant human fibronectin and/or recombinant human vitronectin.

9. The process of any one of the preceding claims, wherein the culturing conditions of step (c) include the use of a xeno- and serum-free culture medium comprising purified native or recombinant human serum albumin, and wherein the xeno- and serum-free culture medium used in step (c) is further **characterized in that** it comprises no EGF.

10. The process of any one of the preceding claims, wherein step (c) comprises passaging the cell population emerged in step (b) at least twice.

11. The process of any one of the preceding claims, further comprising the steps of
(c1) harvesting the cell population obtained in step (b) or after any passage of step (c);
(c2) adding a cryoprotectant to the cell population harvested in step (c1) to obtain a cryoprotected cell formulation;
(c3) freezing said cryoprotected cell formulation; and
(c4) thawing said frozen cryoprotected cell formulation;
wherein steps (c1) to (c4) are performed as sub-steps of step (c).

12. The use of a xeno- and serum-free culture medium comprising purified native or recombinant human serum albumin for culturing primary liver cells obtained from a human liver such as to produce human allogeneic liver-derived cells.

13. The use of a xeno- and serum-free culture medium according to claim 12, wherein the medium is further **characterized in that** it contains less than 30 ng/mL of epidermal growth factor (EGF).

14. The use of a xeno- and serum-free culture medium according to claims 12 or 13, wherein the xeno- and serum-free culture medium further comprises lipids, preferably selected from one or more fatty acids, and wherein the lipids are associated with or bound to the purified native or recombinant human serum albumin.

15. The use of a xeno- and serum-free culture medium according to claims 12 to 14, wherein the the xeno- and serum-free culture medium comprises:
(i) a purified native or recombinant human transferrin, and wherein the human transferrin has optionally been pre-treated with an iron compound;
(ii) recombinant human insulin; and
(iii) a selenium compound, such as sodium selenite.

16. The use of a xeno- and serum-free culture medium according to claims 12 to 15, wherein the xeno- and serum-free culture medium comprises basic fibroblast growth factor (FGF2) at a level of 30 pg/mL or higher, and optionally acidic fibroblast growth factor (FGF1) at a level of 5 pg/mL or higher.

17. The use of a xeno- and serum-free culture medium according to claims 12 to 16, wherein the xeno- and serum-free culture medium comprises human fibronectin and/or human vitronectin, such as recombinant human fibronectin and/or recombinant human vitronectin.

18. An isolated population of human allogeneic liver-derived progenitor cells that are positive for CD90, CD73, vimentin and ASMA; and that exhibit in average less than 2.5% clonal aberrations per metaphase and/or less than 15% non-clonal aberrations per metaphase.

19. The isolated population of human allogeneic liver-derived progenitor cells according to claim 18 for use in the treatment of a liver disease.

20. A cryoprotected cell formulation comprising the isolated population of human allogeneic liver-derived progenitor cells according to claim 18 and one or more cryoprotectants, wherein the cryoprotected cell formulation is optionally frozen.

## Patentansprüche

1. In-vitro-Prozess für die Herstellung einer Population menschlicher, allogener, von der Leber abgeleiteter Stammzellen (HALPC), folgende Schritte umfassend:
(a) Bereitstellen einer Suspension von primären Leberzellen, die von einer menschlichen Leber gewonnen werden,
(b) Kultivieren der primären Leberzellen, die in der Suspension enthalten sind, bis zum Entstehen einer Population von Zellen mit einer länglichen Form und einer mesenchymalen Morphologie, und
(c) Kultivieren der in Schritt (b) entstandenen Zellpopulation unter Bedingungen, die deren Expansion bewirken,
(d) Ernten der in Schritt (c) gewonnenen expandierten Zellpopulation,
wobei die Zellen der in Schritt (c) gewonnenen expandierten Zellpopulation menschliche, allogene, von der Leber abgeleitete Stammzellen (HALPC) sind, die mindestens einen mesenchymalen Marker exprimieren, der aus CD90, CD44, CD73, CD13, CD140b, CD29, Vimentin und alpha-Aktin des glatten Muskels (ASMA) ausgewählt ist, und optional mindestens einen Lebermarker exprimieren und/oder eine leberspezifische Aktivität aufweisen, und
wobei die Kultivierungsbedingungen von Schritt (b) das Verwenden eines xeno- und serumfreien Kulturmediums beinhaltet, das gereinigtes natives oder rekombinantes menschliches Serumalbumin umfasst.

2. Verfahren nach Anspruch 1, wobei das xeno- und serumfreie Kulturmedium ferner **dadurch gekennzeichnet ist, dass** es weniger als 30 ng/ml epidermalen Wachstumsfaktor (EGF) enthält.

3. Verfahren nach Anspruch 1 oder Anspruch 2, ferner den folgenden Schritt umfassend:
(e) Feststellen, dass die Zellen der in Schritt (c) gewonnenen expandierten Zellpopulation menschliche, allogene, von der Leber abgeleitete Stammzellen (HALPC) sind, die mindestens einen mesenchymalen Marker exprimieren, der aus CD90, CD44, CD73, CD13, CD140b, CD29, Vimentin und alpha-Aktin des glatten Muskels (ASMA) ausgewählt ist, und optional mindestens einen Lebermarker exprimieren und/oder eine leberspezifische Aktivität aufweisen.

4. Verfahren nach einem der vorhergehenden Ansprüche, wobei die in Schritt (c) oder (d) gewonnenen menschlichen, allogenen, von der Leber abgeleitete Stammzellen positiv für CD90, CD73, Vimentin und ASMA sind.

5. Verfahren nach einem der vorhergehenden Ansprüche, wobei das xeno- und serumfreie Kulturmedium Lipide umfasst, vorzugsweise aus einer oder mehreren Fettsäuren ausgewählt, und wobei die Lipide mit dem gereinigten nativen oder rekombinanten menschlichen Serumalbumind assoziiert oder daran gebunden sind.

6. Verfahren nach einem der vorhergehenden Ansprüche, wobei das xeno- und serumfreie Kulturmedium Folgendes umfasst:
(i) ein gereinigtes natives oder rekombinantes menschliches Transferrin und wobei das menschliche Transferrin optional mit einer Eisenverbindung vorbehandelt wurde,
(ii) rekombinantes menschliches Insulin und
(iii) eine Selenverbindung wie beispielsweise Natriumselenit.

7. Verfahren nach einem der vorhergehenden Ansprüche, wobei das xeno- und serumfreie Kulturmedium basischen Fibroplast-Wachstumsfaktor (FGF2) auf einem Niveau von 30 pg/ml oder höher und optional sauren Fibroplast-Wachstumsfaktor (FGF1) auf einem Niveau von 5 pg/ml oder höher umfasst.

8. Verfahren nach einem der vorhergehenden Ansprüche, wobei das xeno- und serumfreie Kulturmedium menschliches Fibronectin und/oder menschliches Vitronectin umfasst, wie beispielsweise rekombinantes menschliches Fibronectin und/oder rekombinantes menschliches Vitronectin.

9. Verfahren nach einem der vorhergehenden Ansprüche, wobei die Kultivierungsbedingungen in Schritt (c) die Verwendung eines xeno- und serumfreien Kulturmediums einschließen, das gereinigtes natives oder rekombinantes menschliches Serumalbumin umfasst, und wobei das in Schritt (c) verwendete xeno- und serumfreie Kulturmedium ferner **dadurch gekennzeichnet ist, dass** es keinen EGF umfasst.

10. Verfahren nach einem der vorhergehenden Ansprüche, wobei Schritt (c) das mindestens zweimalige Passagieren der in Schritt (b) entstandenen Zellpopulation umfasst.

11. Verfahren nach einem der vorhergehenden Ansprüche, ferner die folgenden Schritte umfassend:
(c1) Ernten der Zellpopulation, die in Schritt (b) gewonnen wurde, oder nach jeder Passagierung von Schritt (c),
(c2) Zusetzen eines Gefrierschutzmittels zur in Schritt (C1) geernteten Zellpopulation, um eine gefriergeschützte Zellformulierung zu gewinnen,
(c3) Tiefkühlen der gefriergeschützten Zellformulierung und
(c4) Auftauen der tiefgekühlten gefriergeschützten Zellformulierung,
wobei die Schritte (c1) bis (c4) als Teilschritte von Schritt (c) ausgeführt werden.

12. Verwendung eines xeno- und serumfreien Kulturmediums, das gereinigtes natives oder rekombinantes menschliches Serumalbumin umfasst, zum Kultivieren primärer Leberzellen, die von einer menschlichen Leber gewonnen wurden, um beispielsweise menschliche, allogene, von der Leber abgeleitete Zellen zu erzeugen.

13. Verwendung eines xeno- und serumfreien Kulturmediums nach Anspruch 12, wobei das Medium ferner **dadurch gekennzeichnet ist, dass** es weniger als 30 ng/ml epidermalen Wachstumsfaktor (EGF) enthält.

14. Verwendung eines xeno- und serumfreien Kulturmediums nach Anspruch 12 oder 13, wobei das xeno- und serumfreie Kulturmedium ferner Lipide umfasst, vorzugsweise aus einer oder mehreren Fettsäuren ausgewählt, und wobei die Lipide mit dem gereinigten nativen oder rekombinanten menschlichen Serumalbumind assoziiert oder daran gebunden sind.

15. Verwendung eines xeno- und serumfreien Kulturmediums nach den Ansprüchen 12 bis 14, wobei das xeno- und serumfreie Kulturmedium Folgendes umfasst:
(i) ein gereinigtes natives oder rekombinantes menschliches Transferrin und wobei das menschliche Transferrin optional mit einer Eisenverbindung vorbehandelt wurde,
(ii) rekombinantes menschliches Insulin und
(iii) eine Selenverbindung, wie beispielsweise Natriumselenit.

16. Verwendung eines xeno- und serumfreien Kulturmediums nach den Ansprüchen 12 bis 15, wobei das xeno- und serumfreie Kulturmedium basischen Fibroplast-Wachstumsfaktor (FGF2) auf einem Niveau von 30 pg/ml oder höher und optional sauren Fibroplast-Wachstumsfaktor (FGF1) auf einem Niveau von 5 pg/ml oder höher umfasst.

17. Verwendung eines xeno- und serumfreien Kulturmediums nach den Ansprüchen 12 bis 16, wobei das xeno- und serumfreie Kulturmedium menschliches Fibronectin und/oder menschliches Vitronectin umfasst, wie beispielsweise rekombinantes menschliches Fibronectin und/oder rekombinantes menschliches Vitronectin.

18. Isolierte Population menschlicher, allogener, von der Leber abgeleiteter Stammzellen, die positiv für CD90, CD73, Vimentin und ASMA sind und die durchschnittlich weniger als 2,5 % klonale Aberrationen pro Metaphase und/oder weniger als 15 % nichtklonale Aberrationen pro Metaphase aufweisen.

19. Isolierte Population menschlicher, allogener, von der Leber abgeleiteter Stammzellen nach Anspruch 18 zur Behandlung einer Lebererkrankung.

20. Gefriergeschützte Zellformulierung, die isolierte Population menschlicher, allogener, von der Leber abgeleiteter Stammzellen nach Anspruch 18 und ein oder mehrere Gefrierschutzmittel umfassend, wobei die gefriergeschützte Zellformulierung optional tiefgekühlt ist.

## Revendications

1. Procédé in vitro pour la fabrication d'une population de cellules progénitrices humaines allogéniques dérivées du foie (HALPC), comprenant les étapes consistant à :
(a) fournir une suspension de cellules hépatiques primaires obtenues à partir d'un foie humain ;
(b) cultiver les cellules hépatiques primaires comprises dans ladite suspension jusqu'à l'émergence d'une population de cellules ayant une forme allongée et une morphologie mésenchymateuse ;
(c) cultiver la population cellulaire obtenue à l'étape b) dans des conditions qui provoquent son expansion ;
(d) récolter la population cellulaire élargie obtenue à l'étape (c) ;
dans lequel lesdites cellules de la population cellulaire élargie obtenue à l'étape (c) sont des cellules progénitrices humaines allogéniques dérivées du foie (HALPC) qui expriment au moins un marqueur mésenchymateux choisi parmi CD90, CD44, CD73, CD13, CD140b, CD29, la vimentine et l'alpha actine du muscle lisse (ASMA), et qui expriment éventuellement au moins un marqueur hépatique et/ou présentent une activité hépato-spécifique ; et
dans lequel les conditions de culture de l'étape (b) comprennent l'utilisation d'un milieu de culture exempt de xéno et de sérum comprenant de la sérumalbumine humaine native ou recombinante purifiée.

2. Procédé selon la revendication 1, dans lequel le milieu de culture exempt de xéno et de sérum est en outre **caractérisé par le fait qu'**il contient moins de 30 ng/mL de facteur de croissance épidermique (EGF).

3. Procédé selon la revendication 1 ou de la revendication 2, comprenant en outre une étape consistant à
(e) établir que lesdites cellules de la population cellulaire élargie obtenue à l'étape c) sont des cellules progénitrices humaines allogéniques dérivées du foie (HALPC) qui expriment au moins un marqueur mésenchymateux choisi parmi CD90, CD44, CD73, CD13, CD140b, CD29, la vimentine et l'alpha actine du muscle lisse (ASMA), et expriment éventuellement au moins un marqueur hépatique et/ou présentent une activité hépato-spécifique.

4. Procédé selon l'une quelconque des revendications précédentes, dans lequel les cellules progénitrices humaines allogéniques dérivées du foie obtenues à l'étape (c) ou (d) sont positives pour CD90, CD73, vimentine et ASMA.

5. Procédé selon l'une quelconque des revendications précédentes, dans lequel le milieu de culture exempt de xéno et de sérum comprend des lipides, de préférence choisis parmi un ou plusieurs acides gras, et dans lequel les lipides sont associés ou liés à la sérumalbumine humaine native ou recombinante purifiée.

6. Procédé selon l'une quelconque des revendications précédentes, dans lequel le milieu de culture exempt de xéno et de sérum comprend :
(i) une transferrine humaine native ou recombinante purifiée, et dans lequel la transferrine humaine a éventuellement été prétraitée avec un composé de fer ;
(ii) de l'insuline humaine recombinante ; et
(iii) un composé de sélénium, tel que le sélénite de sodium.

7. Procédé selon l'une quelconque des revendications précédentes, dans lequel le milieu de culture exempt de xéno et de sérum comprend un facteur de croissance de fibroblastes basique (FGF2) à un niveau de 30 pg/mL ou plus, et éventuellement un facteur de croissance de fibroblastes acide (FGF1) à un niveau de 5 pg/mL ou plus.

8. Procédé selon l'une quelconque des revendications précédentes, dans lequel le milieu de culture exempt de xéno et de sérum comprend de la fibronectine humaine et/ou de la vitronectine humaine, telle que de la fibronectine humaine recombinante et/ou de la vitronectine humaine recombinante.

9. Procédé selon l'une quelconque des revendications précédentes, dans lequel les conditions de culture de l'étape (c) comportent l'utilisation d'un milieu de culture exempt de xéno et de sérum comprenant de la sérumalbumine humaine native ou recombinante purifiée, et dans lequel le milieu de culture exempt de xéno et de sérum utilisé à l'étape (c) est en outre **caractérisé par le fait qu'**il ne comprend pas d'EGF.

10. Procédé selon l'une quelconque des revendications précédentes, dans lequel l'étape (c) comprend le fait de faire passer la population cellulaire obtenue à l'étape (b) au moins deux fois.

11. Procédé selon l'une quelconque des revendications précédentes, comprenant en outre les étapes consistant à
(c1) récolter la population cellulaire obtenue à l'étape b) ou après tout passage de l'étape c) ;
(c2) ajouter un cryoprotecteur à la population cellulaire récoltée à l'étape (c1) pour obtenir une formulation cellulaire cryoprotégée ;
(c3) congeler ladite formulation cellulaire cryoprotégée ; et
(c4) décongeler ladite formulation cellulaire cryoprotégée congelée ;
dans lequel les étapes (c1) à (c4) sont réalisées comme des sous-étapes de l'étape (c).

12. Utilisation d'un milieu de culture exempt de xéno et de sérum comprenant de la sérumalbumine humaine native ou recombinante purifiée pour cultiver des cellules hépatiques primaires obtenues à partir d'un foie humain afin de produire des cellules humaines allogéniques dérivées du foie.

13. Utilisation d'un milieu de culture exempt de xéno et de sérum selon la revendication 12, dans laquelle le milieu est en outre **caractérisé par le fait qu'**il contient moins de 30 ng/mL de facteur de croissance épidermique (EGF).

14. Utilisation d'un milieu de culture exempt de xéno et de sérum selon les revendications 12 ou 13, dans laquelle le milieu de culture exempt de xéno et de sérum comprend en outre des lipides, de préférence choisis parmi un ou plusieurs acides gras, et dans lequel les lipides sont associés ou liés à la sérumalbumine humaine native ou recombinante purifiée.

15. Utilisation d'un milieu de culture exempt de xéno et de sérum selon les revendications 12 à 14, dans laquelle le milieu de culture exempt de xéno et de sérum comprend :
(i) une transferrine humaine native ou recombinante purifiée, et dans lequel la transferrine humaine a éventuellement été prétraitée avec un composé de fer ;
(ii) de l'insuline humaine recombinante ; et
(iii) un composé de sélénium, tel que le sélénite de sodium.

16. Utilisation d'un milieu de culture exempt de xéno et de sérum selon les revendications 12 à 15, dans laquelle le milieu de culture exempt de xéno et de sérum comprend un facteur de croissance de fibroblastes basique (FGF2) à un niveau de 30 pg/mL ou plus, et éventuellement un facteur de croissance de fibroblastes acide (FGF1) à un niveau de 5 pg/mL ou plus.

17. Utilisation d'un milieu de culture exempt de xéno et de sérum selon les revendications 12 à 16, dans laquelle le milieu de culture exempt de xéno et de sérum comprend de la fibronectine humaine et/ou de la vitronectine humaine, telle que de la fibronectine humaine recombinante et/ou de la vitronectine humaine recombinante.

18. Population isolée de cellules progénitrices humaines allogéniques dérivées du foie qui sont positives pour CD90, CD73, vimentine et ASMA ; et qui présentent en moyenne moins de 2,5 % d'aberrations clonales par métaphase et/ou moins de 15 % d'aberrations non clonales par métaphase.

19. Population isolée de cellules progénitrices humaines allogéniques dérivées du foie selon la revendication 18 pour une utilisation dans le traitement d'une maladie du foie.

20. Formulation cellulaire cryoprotégée comprenant la population isolée de cellules progénitrices humaines allogéniques dérivées du foie selon la revendication 18 et un ou plusieurs cryoprotecteurs, dans laquelle la formulation cellulaire cryoprotégée est éventuellement congelée.
